(19) Europäisches Patentamt
European Patent Office
Office européen des brevets

(11) Publication number: 0 183 271 B1

(12) EUROPEAN PATENT SPECIFICATION

(45) Date of publication of patent specification: 16.05.90

(51) Int. Cl.[5]: C 07 C 311/02, C 07 C 311/10, C 07 K 5/06, C 07 K 5/08, C 07 D 211/16, C 07 D 211/42, C 07 D 213/34, C 07 D 213/40, C 07 D 215/06, C 07 D 215/36, C 07 D 215/08

(21) Application number: 85115142.3

(22) Date of filing: 29.11.85

# (54) Lysin derivative and proteinase inhibitor.

(30) Priority: 30.11.84 JP 251985/84
15.02.85 JP 26556/85
22.03.85 JP 56153/85
02.07.85 JP 143852/85

(43) Date of publication of application:
04.06.86 Bulletin 86/23

(45) Publication of the grant of the patent:
16.05.90 Bulletin 90/20

(84) Designated Contracting States:
CH DE FR GB LI SE

(56) References cited:
DD-B- 155 954
US-A-4 227 006
CHEMICAL ABSTRACTS, vol. 101, no. 23, 3rd December 1984, page 269, column 1, abstract no. 206584f, Columbus, Ohio, US; J. STUERZEBECHER et al.: "Synthetic inhibitors of serine proteinases. Part 31: inhibitory effect of isomeric compounds of N alpha-arylsulfonylated omega-amidinophenyl-alpha-aminoalkylcarboxylic acid amides on trypsin, plasmin and thrombin", & PHARMAZIE 1984, 39(6), 411-413

(73) Proprietor: Okamoto, Shosuke
15-18, Asahigaoka 3-chome Tarumi-ku
Kobe-shi Hyogo (JP)
(73) Proprietor: SHOWA DENKO KABUSHIKI KAISHA
13-9, Shiba Daimon 1-chome
Minato-ku, Tokyo 105 (JP)

(72) Inventor: Okamoto, Shosuke
15-18, Asahigaoka 3-chome Tarumi-ku
Kobe-shi Hyogo (JP)
Inventor: Okada, Yoshio
542-1, Aza Shimizu Okuradani
Akashi-shi Hyogo (JP)
Inventor: Okunomiya, Akiko
13-2, Nakayamatedori 7-chome Chuo-ku
Kobe-shi Hyogo (JP)
Inventor: Naito, Taketoshi SHOWA DENKO K.K.
Seikagakukenkyusho 24-25, Tamagawa 2-chome
Ota-ku Tokyo (JP)
Inventor: Yamada, Morihiko SHOWA DENKO K.K.
Seikagakukenkyusho 24-25, Tamagawa 2-chome
Ota-ku Tokyo (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 183 271 B1

(56) References cited:
CHEMICAL ABSTRACTS, vol. 100, no. 8, 18th-25th June 1984, page 652, column 1, abstract no. 210409z, Columbus, Ohio, US; G. WAGNER et al.: "Synthesis of N-(N alpha-tosyl-4-amidinophenylalanyl)aminocarboxylic acids, esters and amides as potential inhibitors of serine proteinases", & PHARMAZIE 1984, 39(1), 16-18

CHEMICAL ABSTRACTS, vol. 96, no. 8, 29th March - 12th April 1982, page 757, column 1, abstract no. 104710e, Columbus, Ohio, US; G. WAGNER et al.: "Synthesis of 3-(p- and m-amidinophenyl-3-arylsulfonylaminopropionic acid amide hydroiodides", & PHARMAZIE 1981, 36(9), 607-609

Inventor: Kimura, Yoshio SHOWA DENKO K.K.
Seikagakukenkyusho 24-25, Tamagawa 2-chome
Ota-ku Tokyo (JP)
Inventor: Katsuura, Yasuhiro SHOWA DENKO K.K.
Seikagakukenkyusho 24-25, Tamagawa 2-chome
Ota-ku Tokyo (JP)
Inventor: Suzuki, Hiroshi SHOWA DENKO K.K.
Seikagakukenkyusho 24-25, Tamagawa 2-chome
Ota-ku Tokyo (JP)
Inventor: Ohno, Norio SHOWA DENKO K.K.
Seikagakukenkyusho 24-25, Tamagawa 2-chome
Ota-ku Tokyo (JP)
Inventor: Seki, Yumi SHOWA DENKO K.K.
Seikagakukenkyusho 24-25, Tamagawa 2-chome
Ota-ku Tokyo (JP)

(74) Representative: Strehl, Schübel-Hopf, Groening
Maximilianstrasse 54 Postfach 22 14 55
D-8000 München 22 (DE)

**Description**

## Background of the Invention

1. Field of the Invention

The present invention relates to a novel lysine derivative of the pharmaceutically acceptable salt thereof. More specifically, it relates to an L-lysine derivative having a proteinase inhibition activity (e.g., plasmin inhibition activity) or the pharmaceutical acceptable salt thereof and a proteinase inhibitor containing the same as an essential component.

2. Description of the Related Art

It is well-known in the art that various proteinases are present in human organisms. Examples of such proteinases are plasmin, thrombin, trypsin, kallikrein, and urokinase. As is known, when these proteinases are abnormally activated, various diseases are caused. For example, when abnormally activated plasmin is present in a relatively large amount in the blood, hemorrhagic disorder or inflammatory disorder are caused. For this reason, a substance capable of exhibiting a proteinase inhibition activity is useful as a clinical remedy or medicine.

It as been reported in, for example, J. Biol. Chem. *208,* 85 (1954) and J. Biochem., *57,* 450 (1965) that certain derivatives of lysine and arginine have an inhibition activity against plasmin, which is a proteinase specific to fibrin and fibrinogen in blood. However, the plasmin inhibition activity of the reported substances is low and, therefore, practical use of those substances as a medicine is not acceptable in the art.

## Summary of the Invention

An object of the present invention is to provide a novel compound having an effective proteinase inhibition activity suitable for use as a proteinase inhibitor such as plasmin inhibitor.

Other objects and advantages of the present invention will be apparent from the following description.

In accordance with the present invention, there is provided a lysine derivative having the general formula:

A-Y-Lys-B (L-form) (A)

wherein A represents

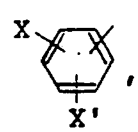, 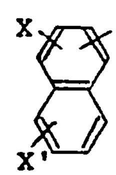, 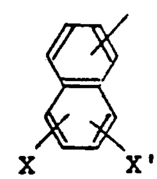,

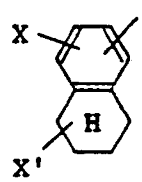, 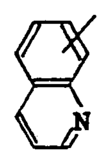, 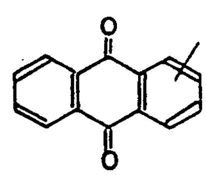, 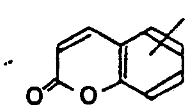,

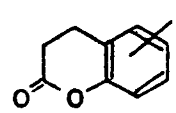, 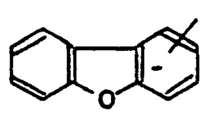, 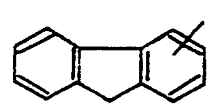,

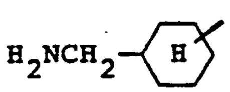, $H_2N(CH_2)_{n_1}$- , 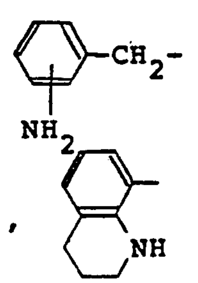,

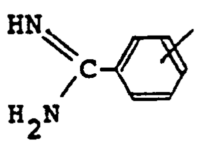, 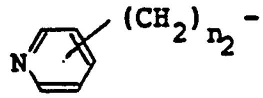,

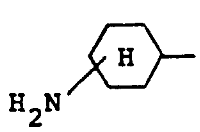, or 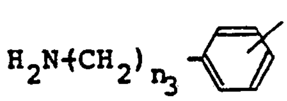;

wherein X and X' independently represent hydrogen, halogen, alkyl preferably having 1 to 5 carbon atoms, cycloalkyl preferably having 5 to 8 carbon atoms, alkoxy preferably having 1 to 5 carbon atoms, aryloxy preferably having 6 to 10 carbon atoms, dialkylamino preferably having a $C_1$ to $C_5$ alkyl group, alkylcarbonylamino preferably having a $C_1$ to $C_5$ alkyl group, arylcarbonylamino preferably having a $C_6$ to $C_{10}$ aryl group, and $n_1$ is an integer of 3 to 6, $n_2$ is an integer of 1 to 3, and $n_3$ is an integer of 0 to 3;

Y represents $SO_2$ or CO;

-Lys- represents

$$\begin{array}{c} (CH_2)_4{-}NH_2 \\ | \\ {-}NH{-}CH{-}CO{-}; \end{array}$$

B represents $NR^1R^2$, NZW, or tetrahydroquinolyl wherein $R^1$ and $R^2$ independently represents hydrogen provided that both $R^1$ and $R^2$ cannot be hydrogen at the same time; alkyl preferably having 1 to 6 carbon atoms substituted with carboxyl, alkoxycarbonyl preferably having 2 to 6 carbon atoms, phenyl, hydroxyphenyl, or benzoyl; cycloalkyl preferably having 5 to 8 carbon atoms, which may be substituted with arylcarbonyl preferably having a $C_6$ to $C_{10}$ aryl group; cycloalkylalkyl preferably having 6 to 11 carbon atoms, which may be substituted with carboxyl, arylcarbonyl preferably having a $C_6$ to $C_{10}$ aryl group, or aralkyloxycarbonyl preferably having a $C_7$ to $C_{11}$ aralkyl group; phenyl which may be substituted with halogen, nitro, cyano, trifluoromethyl, alkyl preferably having 1 to 5 carbon atoms, alkoxy preferably having 1 to 5 carbon atoms, alkoxycarbonyl preferably having 2 to 10 carbon atoms, alkoxycarbonylalkyl preferably having 3 to 10 carbon atoms, phenylalkyl preferably having 7 to 10 carbon atoms which may be further substituted with dialkylamino preferably having a $C_1$ to $C_3$ alkyl group, alkylcarbonyl preferably having a $C_1$ to $C_{10}$ alkyl group, phenylalkenyl preferably having 8 to 10 carbon atoms which may be further substituted with dialkylamino preferably having a $C_1$ to $C_3$ alkyl group, phenoxy, phenylcarbonyl which may be further substituted with amino, dialkylamino preferably having a $C_1$ to $C_3$ alkyl group, alkoxycarbonyl preferably having 2 to 6 carbon atoms, or nitro, pyridylmethyl, phenyl hydroxyalkyl preferably having a $C_1$ to $C_3$ alkyl group, alkylsulfonyl preferably having a $C_1$ to $C_{20}$ alkyl group, or alkoxycarbonyl alkylaminocarbonyl preferably having 4 to 6 carbon atoms; coumaryl which may be sustituted with alkyl preferably having 1 to 5 carbon atoms; quinolyl; adamantyl; norbornyl; or tetrahydronaphthyl; and

Z is

$$\begin{array}{c} | \\ {-}(CH_2)_{\overline{m_1}}CH(CH_2)_{\overline{m_2}} \end{array}$$

or

$$\begin{array}{c} | \\ {-}(CH_2)_{\overline{m_1}}{-}N{-}(CH_2)_{\overline{m_2}}\ ; \end{array}$$

W is hydrogen; hydroxyl; carboxyl; aminocarbonyl; alkyl preferably having 1 to 10 carbon atoms; alkoxycarbonyl preferably having 2 to 10 carbon atoms; phenyl; phenylalkyl preferably having 7 to 12 carbon atoms; atoms which may be substituted with dialkylamino preferably having a $C_1$ to $C_3$ alkyl group; or phenylcarbonyl which may be substituted with alkoxycarbonyl preferably having 7 to 11 carbon atoms; and

$$m_1 + m_2 = 3 \text{ or } 4;$$

or the pharmaceutically acceptable salt thereof.

Examples of the pharmaceutically acceptable salts are inorganic acid salts such as hydrochloride, hydrobromide, sulfate, nitrate, and phosphate and organic acid salts such as oxalate, succinate, malate, citrate, lactate, benzene sulfonate, toluene sulfonate, and methane sulfonate.

In accordance with the present invention, there is also provided a proteinase inhibitor containing as an essential component the above-mentioned L-lysine derivatives or the pharmaceutically acceptable salts thereof.

## Description of the Preferred Embodiments

Typical examples of the L-lysine derivatives according to the present invention are summarized in Table I, wherein (D) indicated under the carbon atom of the compound Nos. 12, 15, and 22 denotes that the carbon atom is in the D-form and Lys, Phe, and Pro in the formula of the compounds represent L-lysine, phenylalanine, and proline, respectively. In the physical properties shown in Table I, NMR represents a nuclear magnetic resonance spectrum indicated by δ (i.e., delta) (ppm) representing the chemical shifts. The determination was carried out by using as a solvent $CDCl_3$ (i.e., heavy chloroform), $(CD_3)_2SO$ (i.e., $d^6$-dimethylsulfoxide), or $CD_3OD$ (i.e., heavy methanol) alone or in any mixture thereof and by using as an internal standard TMS (i.e., tetramethylsilane). In the parenthesis after the δ number, the number of the hydrogen atom and the symbols s, d, t, q, m, and broad thereafter means singlet, doublet, triplet, quartet,

multiplet, and broad absorbance, respectively. The absorbance based on the solvent is deleted from the Table.

IR represents an infrared absorption spectrum in which a potassium bromide tablet is used in the determination unless otherwise noted. When a solution is used in the determination, the kind of the solvent is listed in parenthesis. The number listed in the Table represents a wave number in units of $cm^{-1}$ and only the main absorption peaks are listed in the Table.

MS represents a mass spectrum, and the results are shown as M/e (i.e., the mass of the cation fragment divided by the charge) of the main peaks.

Table I (List of Compounds of Present Invention)

| Compound No. | Compound | Physical Properties |
|---|---|---|
| 1 | 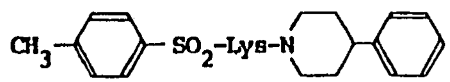 | NMR:<br>$CDCl_3$, TMS<br>δ 1.5 (10H, m)<br>2.3 (3H, s)<br>2.4 (1H, s)<br>2.5 - 4.5 (11H, m)<br>7.1 - 7.9 (9H, m) |
| 2 | 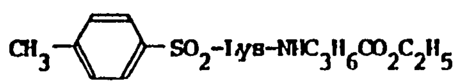 | MS:<br>M/e 413, 395, 368, 356, 326, 312, 255, 241, 238 |
| 3 | 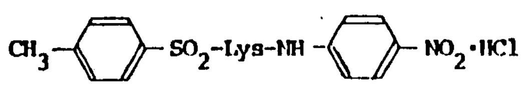 | IR:<br>1690, 1600, 1160, 1310 |

Table I (List of Compounds of Present Invention) (Continued)

| Compound No. | Compound | Physical Properties | |
|---|---|---|---|
| 4 | 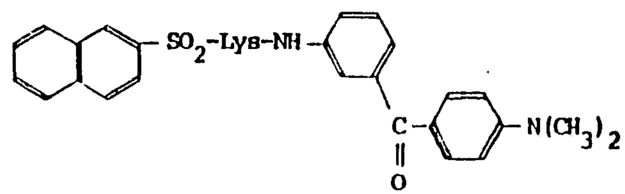 | MS: M/e 541, 399, 318, 267, 240, 207, 176, 160 148, 128 | IR: 3400, 1680, 1580, 1150 |
| 5 | 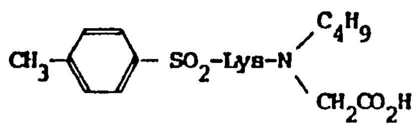 | MS: M/e 356, 312, 283, 255, 238, 226, 183, 171, 155 | IR: 1640, 1340, 1160 |
| 6 | 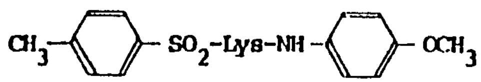 | IR: 1680, 1600, 1320, 1160 | |
| 7 | 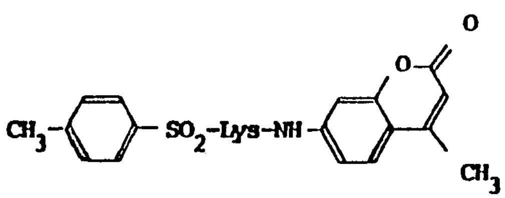 | IR: 1720, 1700, 1660, 1615, 1575, 1520, 1420, 1380, 1320, 1160 | |
| 8 | 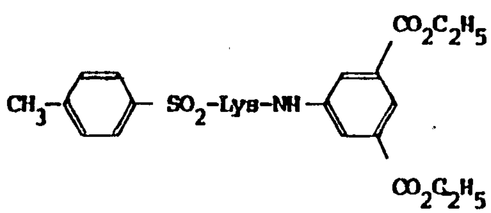 | MS: M/e 518, 500, 464, 455, 374, 359, 345, 342 | IR: 1720, 1670, 1660, 1600, 1540, 1450, 1365, 1320, 1240, 1180, 1160 |

Table I (List of Compounds of Present Invention) (Continued)

| Compound No. | Compound | Physical Properties | |
|---|---|---|---|
| 9 | 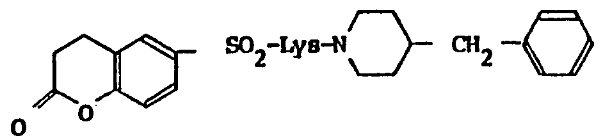 | MS:<br>M/e 284, 339 | NMR:<br>$CDCl_3$ , TMS<br>δ 7.58 - 6.96 (8H, m)<br>2.0 - 3.0 (13H, m)<br>3.3 - 3.6 (1H, t)<br>0.8 - 1.8 (10H, broad) |
| 10 | 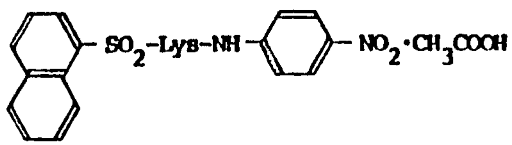 | IR:<br>1695, 1600, 1310, 1160 | |
| 11 | 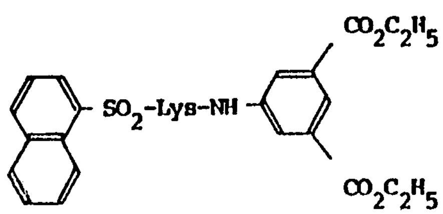 | IR:<br>1700, 1630, 1540, 1450,<br>1320, 1240, 1160, 1130 | NMR:<br>$CDCl_3$ , TMS<br>δ 0.70 - 2.35 (12H, m)<br>2.50 - 3.00 (2H, m)<br>4.0 - 5.20 (4H, m)<br>7.40 - 9.0 (10H, m) |

Table I (List of Compounds of Present Invention) (Continued)

| Compound No. | Compound | Physical Properties | |
|---|---|---|---|
| 12 | 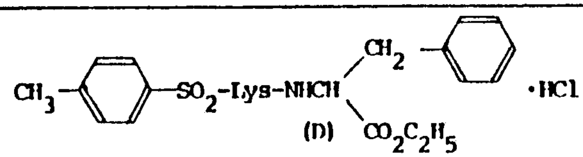 | MS:<br>M/e 475, 303, 256, 120, 84 | NMR:<br>$CDCl_3$ , TMS<br>δ 1.08 - 1.80 (9H, m)<br>2.42 (3H, s)<br>2.60 - 3.20 (4H, m)<br>4.15 (2H, q)<br>7.05 - 7.98 (9H, m) |
| 13 | 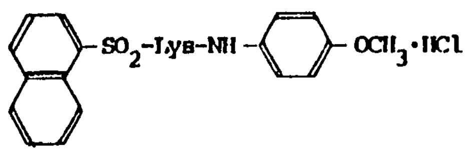 | IR:<br>1660, 1600, 1310, 1160 | |
| 14 | 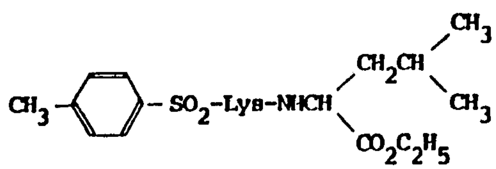 | MS:<br>M/e 441, 426, 398, 396, 368, 269, 255 | |
| 15 | 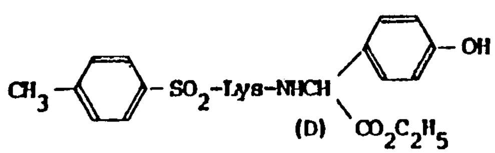 | MS:<br>M/e 477, 432, 378, 350, 333, 305, 303, 283, 255, 231, 179, 171, 155, 127, 91 | IR:<br>1730, 1650, 1325, 1160 |

Table I (List of Compounds of Present Invention) (Continued)

| Compound No. | Compound | Physical Properties | |
|---|---|---|---|
| 16 | 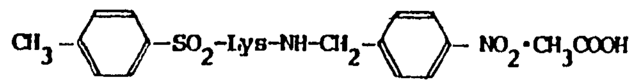 | IR:<br>1650, 1600, 1320, 1160 | |
| 17 | 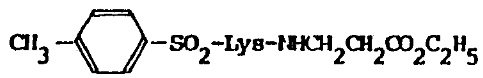 | MS:<br>M/e 354, 255, 227, 155, 84 | IR:<br>1635, 1320, 1160 |
| 18 | 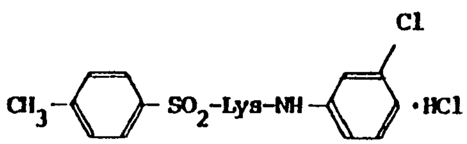 | MS:<br>M/e 255, 237, 155, 127, 84 | NMR:<br>$CD_3OD$, TMS<br>δ 1.08 - 1.95 (6H, broad)<br>2.12 (3H, s)<br>2.80 - 3.15 (2H, broad)<br>7.05 - 7.90 (13H, broad) |
| 19 | 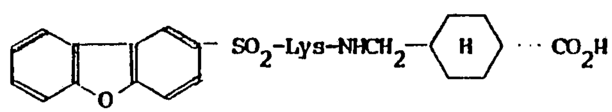 | MS:<br>M/e 358, 247, 231, 200, 183, 168, 157, 127, 84 | IR:<br>3400, 1710, 1640, 1160 |

Table I (List of Compounds of Present Invention) (Continued)

| Compound No. | Compound | Physical Properties | |
|---|---|---|---|
| 20 | 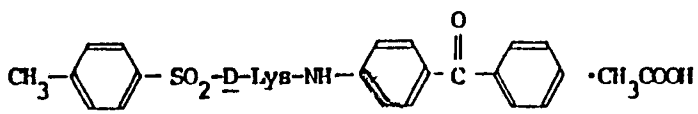 | IR ($CHCl_3$)<br>1690, 1600, 1320, 1160 | |
| 21 | 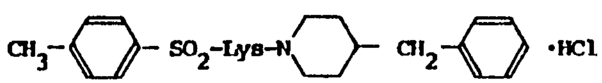 | MS:<br>M/e 457, 339, 385, 285, 283,<br>255, 238, 202, 174, 84 | NMR:<br>Free<br>$CDCl_3$ , TMS<br>δ 1.2 - 2.0 (10H, broad)<br>2.06 - 3.18 (11H, m)<br>3.8 - 4.2 (2H, broad)<br>6.80 - 7.88 (9H, m) |
| 22 | 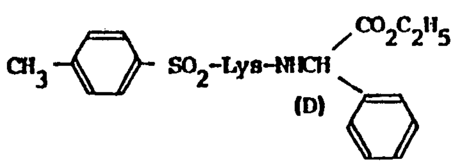 | MS:<br>M/e 461, 416, 388, 290, 255,<br>171, 127, 106, 84 | IR:<br>1740, 1660, 1160 |
| 23 | 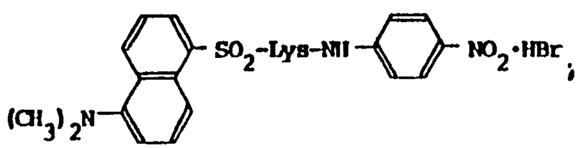 | IR:<br>1700, 1600, 1320, 1160 | |

Table I (List of Compounds of Present Invention) (Continued)

| Compound No. | Compound | Physical Properties | |
|---|---|---|---|
| 24 | 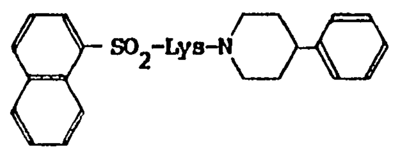 | MS:<br>M/e 479, 407, 291, 271, 191, 127, 84 | NMR:<br>$CDCl_3$-$CD_3OD$, TMS<br>δ 1.35 - 1.90 (10H, broad)<br>2.35 - 3.00 (2H, broad)<br>3.05 - 4.40 (4H, m)<br>6.80 - 8.80 (12H, m) |
| 25 | 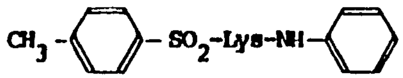 | MS:<br>M/e 375, 248, 203, 155, 93, 84 | NMR:<br>$CDCl_3$ , TMS<br>δ 1.20 - 2.0 (6H, broad)<br>2.20 (3H, s)<br>2.50 - 2.95 (1H, broad)<br>3.47 (6H, broad)<br>6.90 - 8.0 (9H, m) |
| 26 | 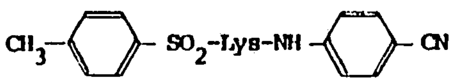 | MS:<br>M/e 278, 255, 246, 227, 156 139, 123, 118, 84 | NMR:<br>$CDCl_3$ , TMS<br>δ 1.20 - 1.95 (6H, broad)<br>2.30 (3H, s)<br>2.45 - 3.02 (2H, broad)<br>3.25 - 4.20 (2H, broad)<br>7.10 - 7.90 (8H, broad) |
| 27 | 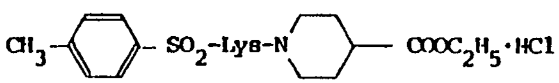 | IR:<br>1721, 1620, 1600, 1300, 1140 | |

Table I (List of Compounds of Present Invention) (Continued)

| Compound No. | Compound | Physical Properties | |
|---|---|---|---|
| 28 | 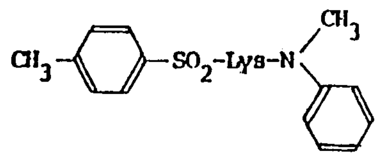 | MS:<br>M/e 309, 255, 238, 217, 155, 107, 84 | IR:<br>3300, 3250, 1665, 1315, 1140 |
| 29 | 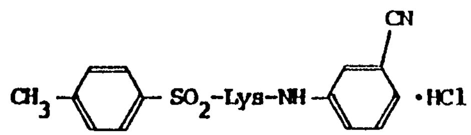 | MS:<br>M/e 400, 382, 343, 317, 278, 255, 246, 238, 227 | |
| 30 | 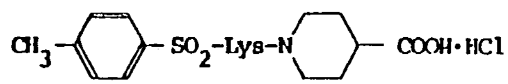 | IR ($CHCl_3$)<br>1700, 1640, 1300, 1160 | |
| 31 | 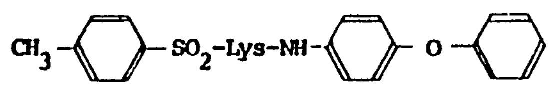 | MS:<br>M/e 467, 312, 293, 255, 185, 155, 127, 84 | IR:<br>1680, 1220, 1155 |
| 32 | 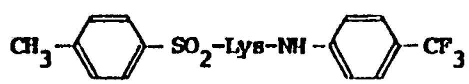 | MS:<br>M/e 425, 427, 333, 271, 255, 209, 161, 84 | NMR:<br>$CDCl_3$ , TMS<br>δ 0.90 - 2.0 (6H, broad)<br>2.10 - 2.95 (5H, m)<br>4.40 - 5.05 (2H, broad)<br>6.90 - 8.10 (8H, m) |

Table I (List of Compounds of Present Invention) (Continued)

| Compound No. | Compound | Physical Properties | |
|---|---|---|---|
| 33 | 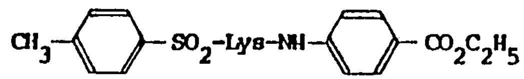 | MS:<br>M/e 274, 255, 209, 165, 127, 120, 110, 84 | |
| 34 | 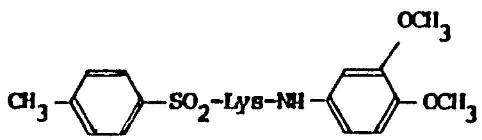 | MS:<br>M/e 435, 263, 231, 153, 84 | NMR:<br>$CDCl_3$, TMS<br>δ 1.08 - 1.88 (6H, broad)<br>2.26 (3H, s)<br>2.58 (2H, m)<br>3.76 (3H, s)<br>3.12 - 4.56 (8H, m)<br>6.56 - 7.84 (7H, m) |
| 35 | 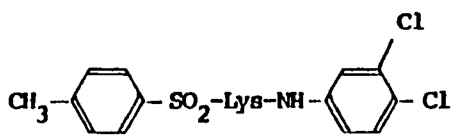 | MS:<br>M/e 443, 271, 255, 161, 155 127, 84 | IR:<br>1680, 1590, 1480, 1160, 820 |
| 36 | 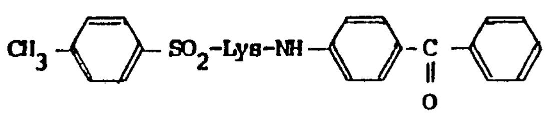 | IR:<br>1660, 1600, 1315, 1160 | NMR:<br>$CDCl_3$, TMS<br>δ 1.10 - 1.82 (6H, broad)<br>2.21 (3H, s)<br>2.50 - 2.75 (2H, m)<br>3.90 - 4.05 (2H, m)<br>6.90 - 7.85 (13H, m) |

Table I (List of Compounds of Present Invention) (Continued)

| Compound No. | Compound | Physical Properties | |
|---|---|---|---|
| 37 | 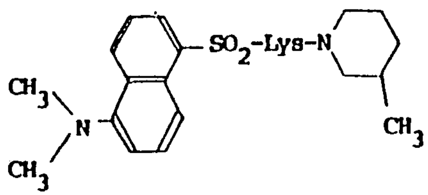 .HCl | IR: ($CHCl_3$)<br>1640, 1600, 1340, 1160 | |
| 38 | 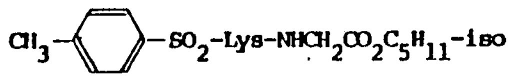 | MS:<br>M/e 255, 238, 226 | IR:<br>1745, 1640, 1160 |
| 39 | 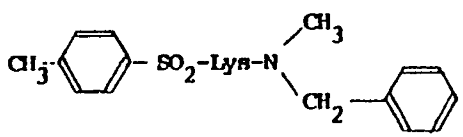 .HCl | NMR:<br>Free<br>$CDCl_3$ , TMS<br>δ 1.10 - 1.80 (6H, broad)<br>2.40 (3H, s)<br>2.72 (3H, s)<br>2.60 - 2.85 (2H, broad)<br>6.90 - 7.85 (9H, m) | |
| 40 | 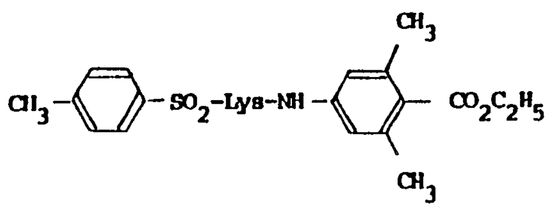 | MS:<br>M/e 475, 430, 320, 303, 255<br>193, 155, 148, 84 | IR:<br>1710, 1640, 1160 |

Table I (List of Compounds of Present Invention) (Continued)

| Compound No. | Compound | Physical Properties | |
|---|---|---|---|
| 41 | 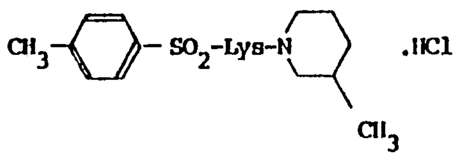 | IR ($CHCl_3$):<br>1640, 1600, 1342, 1160 | |
| 42 | 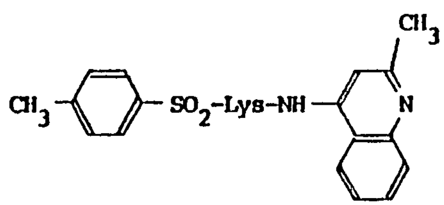 | MS:<br>M/e 267, 255, 185, 158, 155, 127, 84 | IR:<br>1685, 1520, 1320, 1160 |
| 43 | 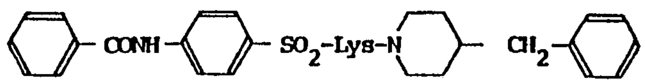 | MS:<br>M/e 544, 490, 360, 285, 276, 260, 203, 197, 174, 105, 84 | NMR:<br>$CDCl_3$, TMS<br>δ 1.12 - 2.08 (10H, broad)<br>2.16 - 2.56 (2H, broad)<br>2.60 - 4.04 (7H, broad)<br>6.80 - 8.60 (14H, m) |
| 44 | 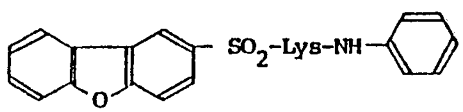 | MS:<br>M/e 358, 331, 247, 231, 200, 167, 127, 110, 84 | IR:<br>3550, 1740, 1160, 750 |

Table I (List of Compounds of Present Invention) (Continued)

| Compound No. | Compound | Physical Properties | |
|---|---|---|---|
| 45 | 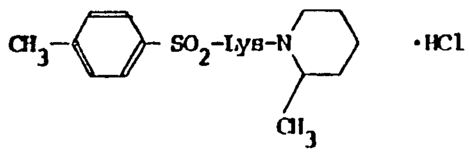 | | IR:<br>1640, 1600, 1330, 1160 |
| 46 | 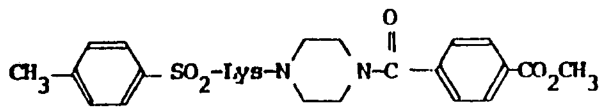 | MS:<br>M/e 403, 358, 356, 283, 171, 163, 155 | IR:<br>1720, 1630, 1430, 1280, 1160 |
| 47 | 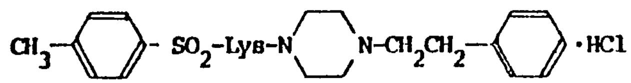 | MS:<br>M/e 472, 407, 381, 362, 255, 155, 84 | NMR:<br>$CDCl_3$, TMS<br>δ 1.30 - 2.05 (8H, m)<br>2.15 (3H, s)<br>2.30 - 3.65 (15H, m)<br>6.20 - 6.90 (9H, m) |
| 48 | 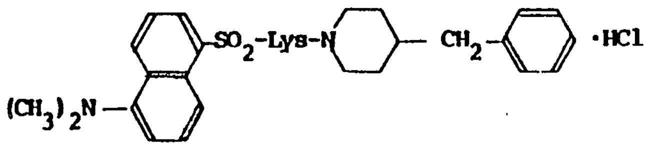 | MS:<br>M/e 536, 463, 361, 334, 316, 285, 251, 234, 174, 170, 84 | IR:<br>Free<br>1630, 1320, 1140 |
| 49 | 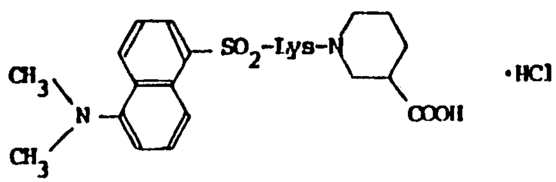 | IR ($CHCl_3$)<br>1710, 1640, 1335, 1160 | |

Table I (List of Compounds of Present Invention) (Continued)

| Compound No. | Compound | Physical Properties | |
|---|---|---|---|
| 50 | 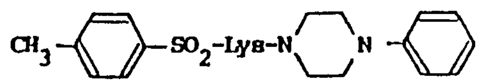 | MS: M/e 444, 326, 312, 272, 255, 189, 161, 145, 132, 119, 84 | IR: 1640, 1590, 1310, 1140 |
| 51 | 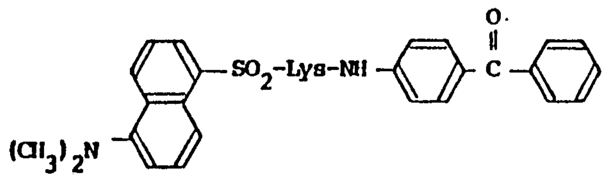 | MS: M/e 317, 250, 235, 197, 171, 127, 80 | IR: 1690, 1640, 1590, 1140 |
| 52 | 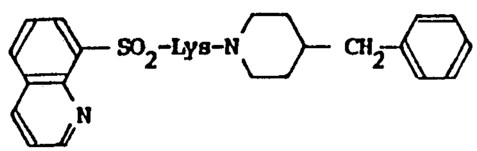 | MS: M/e 422, 292, 285, 209, 192, 174, 129, 84 | IR: 1635, 1335, 1170, 1145 |
| 53 | 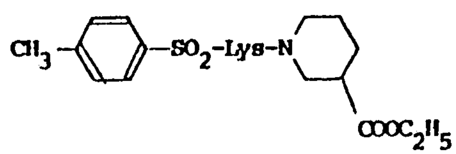 .HCl | IR ($CHCl_3$) 1724, 1644, 1340, 1160 | |
| 54 | 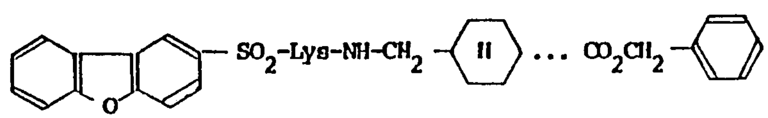 | MS: M/e 358, 331, 247, 231, 200, 168, 127, 91, 84 | IR: 3400, 1720, 1660, 1150 |

Table I (List of Compounds of Present Invention) (Continued)

| Compound No. | Compound | Physical Properties | |
|---|---|---|---|
| 55 | 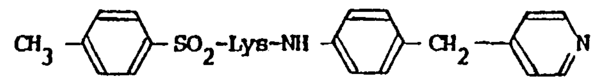 | MS: M/e 466, 293, 282, 255, 211, 184, 127, 106, 84 | IR: 1670, 1600, 1155 |
| 56 | 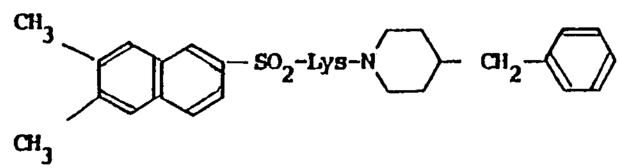 | MS: M/e 521, 449, 401, 319, 285, 235, 219, 174, 155, 84 | IR: 1630, 1150 |
| 57 | 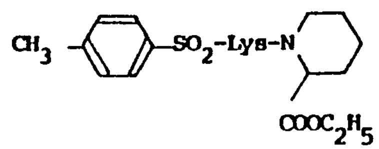 .HCl | IR: 1722, 1645, 1600, 1335, 1160 | |
| 58. | 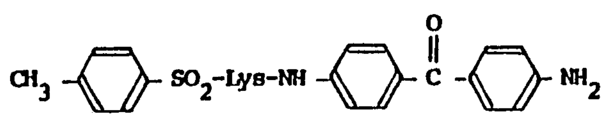 | MS: M/e 335, 321, 239, 212, 171, 156, 139, 124, 120, 92, 91 | |
| 59 | 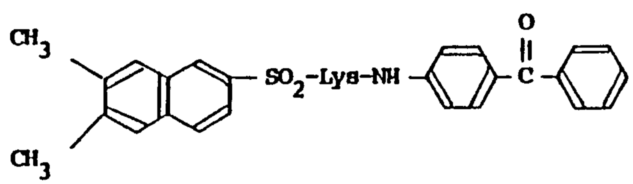 | MS: M/e 544, 374, 235, 197, 188, 155, 91, 80 | IR: 1690, 1585, 1150 |

Table I (List of Compounds of Present Invention) (Continued)

| Compound No. | Compound | Physical Properties | |
|---|---|---|---|
| 60 | 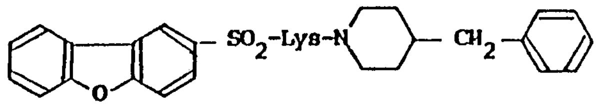 | MS:<br>M/e 533, 413, 331, 285, 247, 231, 174, 167, 84 | IR:<br>1680, 1620, 1160 |
| 61 | 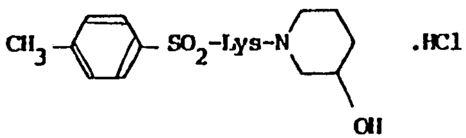 | IR:<br>1650, 1600, 1330, 1160 | |
| 62 | 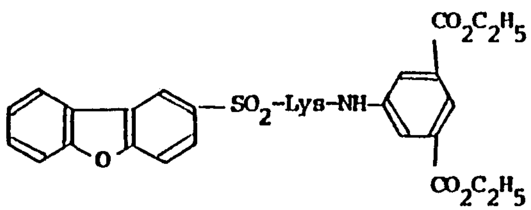 | MS:<br>M/e 345, 237, 231, 168, 80 | IR:<br>1720, 1150 |
| 63 | 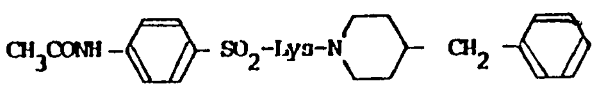 | MS:<br>M/e 527, 482, 456, 440, 428, 386, 340, 298, 256, 198, 174, 126, 93, 84 | NMR:<br>$(CD_3O)_2SO$, TMS<br>δ 1.0 - 1.80 (10H, broad)<br>2.14 (3H, s)<br>2.20 - 3.88 (8H, broad)<br>7.80 - 8.20 (9H, m) |

Table I (List of Compounds of Present Invention) (Continued)

| Compound No. | Compound | Physical Properties | |
|---|---|---|---|
| 64 | 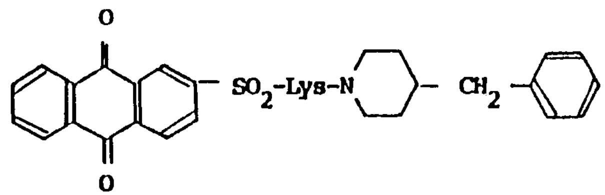 | MS:<br>M/e 446, 354, 285, 240, 208<br>180, 174, 152, 101, 84 | |
| 65 | 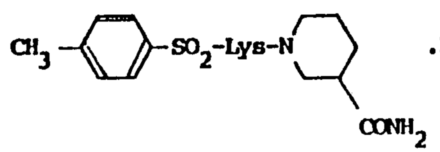 .HCl | IR:<br>1670, 1630, 1598, 1320, 1160 | |
| 66 | 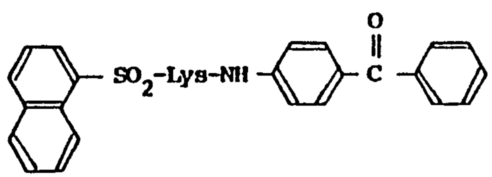 | MS:<br>M/e 396, 318, 223, 207, 197,<br>192, 176, 160, 128 | NMR:<br>$CDCl_3$, TMS<br>δ 1.0 - 1.88 (6H, m)<br>2.60 (2H, broad)<br>3.00 - 4.10 (3H, m)<br>6.96 - 9.04 (16H, m) |
| 67 | 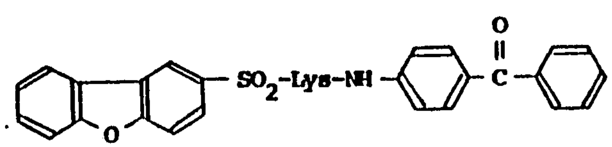 | MS:<br>M/e 291, 247, 231, 200, 168,<br>84 | IR:<br>1690, 1650, 1590, 1310, 1150 |
| 68 | 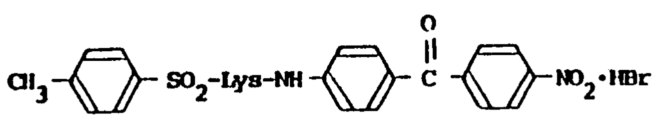 | MS:<br>M/e 350, 246, 171, 155, 120,<br>91, 80 | IR:<br>1680, 1650, 1590, 1525,<br>1270, 1155 |

Table I (List of Compounds of Present Invention) (Continued)

| Compound No. | Compound | Physical Properties | |
|---|---|---|---|
| 69 | 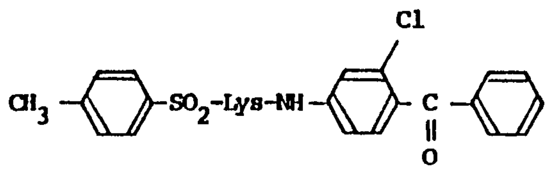 | MS:<br>M/e 340, 231, 154 | NMR:<br>$CDCl_3$ , TMS<br>δ 1.20 - 1.92 (6H, broad)<br>2.36 (3H, s)<br>2.70 (3H, d)<br>3.04 - 3.84 (3H, broad)<br>3.96 (1H, m)<br>7.16 - 8.84 (12H, m) |
| 70 | 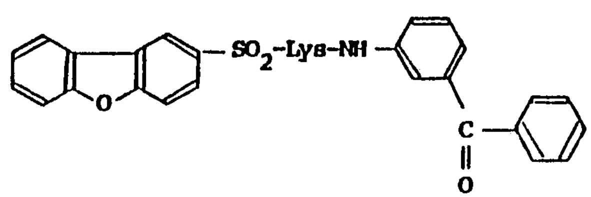 | MS:<br>M/e 314, 291, 247, 232, 216, 200, 197, 183, 168, 80 | IR:<br>1690, 1650, 1585, 1310, 1150 |
| 71 | 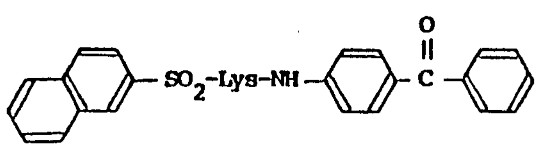 | MS:<br>M/e 291, 274, 207, 197, 160, 128, 80 | IR:<br>1680, 1640, 1585, 1150 |

Table I (List of Compounds of Present Invention) (Continued)

| Compound No. | Compound | Physical Properties | |
|---|---|---|---|
| 72 | 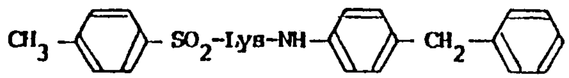 | MS:<br>M/e 367, 292, 263, 201, 183, 155, 106, 91, 83 | NMR:<br>$CDCl_3$ , TMS<br>δ 1.15 - 1.88 (6H, m)<br>2.28 (3H, s)<br>2.48 - 3.24 (2H, m)<br>3.90 (3H, m)<br>6.88 - 8.68 (13H, m) |
| 73 | 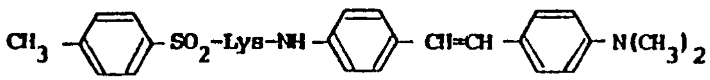 | MS:<br>M/e 503, 347, 264, 238, 223, 222, 171, 155, 139, 91 | NMR:<br>$CDCl_3$ , TMS<br>δ 1.16 - 1.84 (6H, broad)<br>1.84 - 2.44 (3H, broad)<br>2.50 - 3.10 (2H, m)<br>2.96 (6H, s)<br>6.60 - 6.95 (2H, m)<br>7.0 - 7.88 (12H, m) |
| 74 | 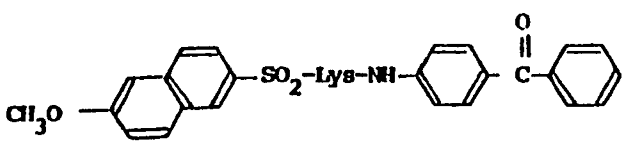 | IR:<br>3400, 1685, 1640, 1590, 1150 | NMR:<br>$CDCl_3$ , TMS<br>δ 1.10 - 1.84 (6H, broad)<br>2.60 - 2.84 (2H, broad)<br>3.88 (3H, s)<br>6.80 - 8.70 (15H, m) |

Table I (List of Compounds of Present Invention) (Continued)

| Compound No. | Compound | Physical Properties | |
|---|---|---|---|
| 75 | 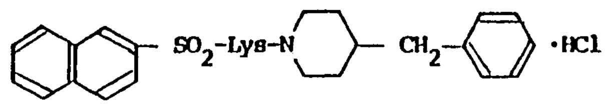 | MS:<br>M/e 493, 475, 421, 291, 285, 274, 191, 174, 127 | NMR:<br>$CDCl_3$ , TMS<br>δ 0.68 - 2.04 (10H, m)<br>2.06 - 2.80 (3H, m)<br>3.0 - 4.32 (6H, m)<br>6.72 - 8.60 (12H, m) |
| 76 | 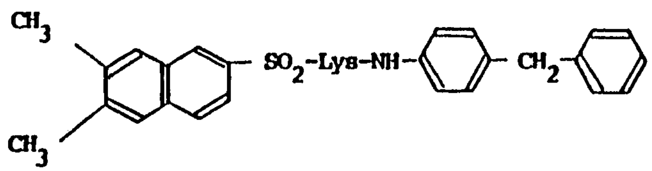 | MS:<br>M/e 444, 374, 346, 302, 292, 235, 220, 209, 204, 180, 156, 106, 83 | NMR:<br>$CDCl_3$-$CD_3OD$, TMS<br>δ 1.12 - 1.88 (6H, m)<br>2.12 - 2.92 (8H, m)<br>3.84 (3H, broad)<br>6.72 - 8.40 (14H, m) |
| 77 | 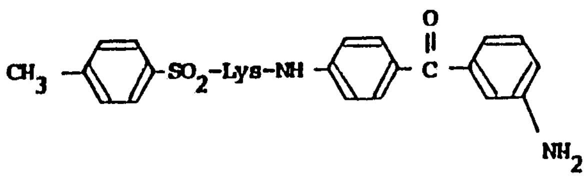 | MS:<br>M/e 323, 255, 246, 238, 171, 156, 139, 124, 108, 92, 84 | |
| 78 | 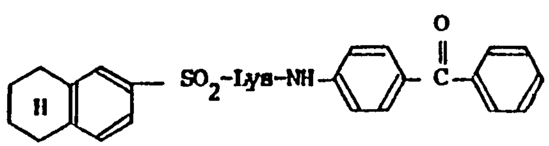 | MS:<br>M/e 291, 211, 197, 196, 183, 164, 131, 84 | IR:<br>1680, 1650, 1590, 1150 |

Table I (List of Compounds of Present Invention) (Continued)

| Compound No. | Compound | Physical Properties | |
|---|---|---|---|
| 79 | 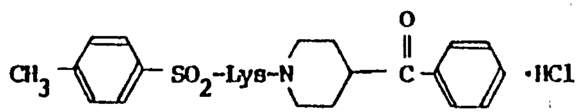 | MS:<br>M/e 255, 238, 226, 173, 156, 91, 84 | IR:<br>3320, 2940, 2900, 2840, 1670, 1630, 1440, 1320, 1155 |
| 80 | 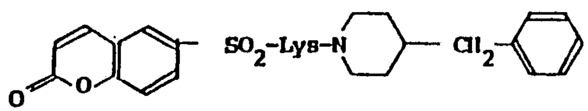 | MS:<br>M/e 493, 336, 309, 285, 225, 209, 175, 146, 118, 84 | NMR:<br>$CDCl_3$, TMS<br>δ 1.20 - 2.16 (10H, m)<br>2.24 - 3.08 (6H, m)<br>3.40 - 3.80 (1H, broad)<br>3.96 - 4.36 (1H, broad)<br>6.40 - 8.20 (10H, m) |
| 81 | 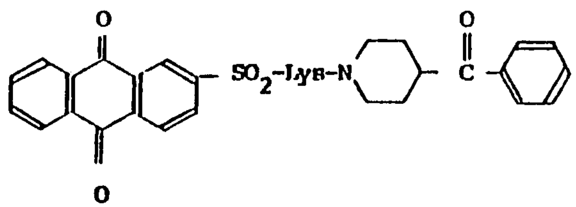 | MS:<br>M/e 298, 287, 240, 208, 180, 152, 84, 64 | IR:<br>1675, 1635, 1585, 1440, 1320, 1285, 1175, 1150 |
| 82 | 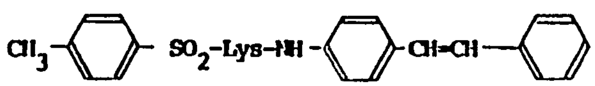 | MS:<br>M/e 304, 195, 127, 110, 99, 91, 83 | NMR:<br>$CDCl_3$, TMS<br>δ 1.24 - 1.88 (6H, m)<br>2.28 (3H, s)<br>2.40 - 3.0 (6H, broad)<br>3.88 (1H, m)<br>6.98 - 7.82 (15H, m) |

Table I (List of Compounds of Present Invention) (Continued)

| Compound No. | Compound | Physical Properties | |
|---|---|---|---|
| 83 | 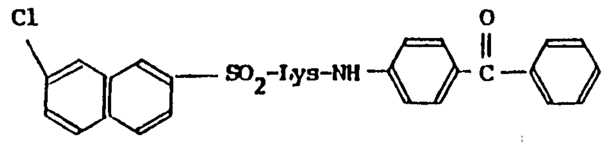 | MS:<br>M/e 308, 291, 241, 225, 197, 194, 162, 127 | IR:<br>1690, 1645, 1600, 1160 |
| 84 | 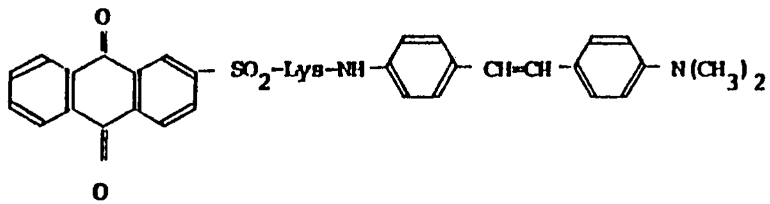 | MS:<br>M/e 347, 287, 264, 254, 238, 208, 194, 120, 84 | IR:<br>1670, 1600, 1155 |
| 85 | 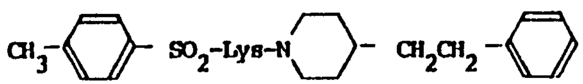 | MS:<br>M/e 343, 321, 297, 188, 171, 155, 91, 84 | |
| 86 | 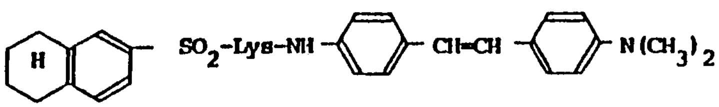 | MS:<br>M/e 347, 264, 238, 223, 211, 196, 179, 164, 131, 105, 91, 84 | |
| 87 | 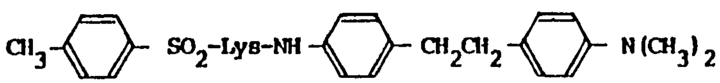 | MS:<br>M/e 283, 255, 240, 226, 156, 134, 84 | |

Table I (List of Compounds of Present Invention) (Continued)

| Compound No. | Compound | Physical Properties | |
|---|---|---|---|
| 88 | 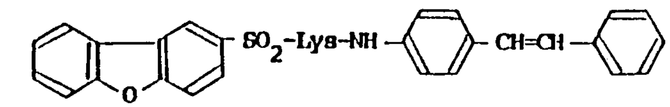 | MS:<br>M/e 398, 350, 304, 200, 195, 168 | |
| 89 | 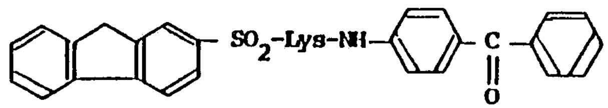 | MS:<br>M/e 288, 230, 198, 197, 165, 120, 80 | IR:<br>1690, 1640, 1150 |
| 90 | 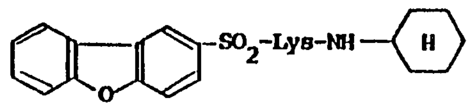 | MS:<br>M/e 457, 358, 331, 247, 231, 209, 167, 127, 84 | IR:<br>3400, 1640, 1150 |
| 91 | 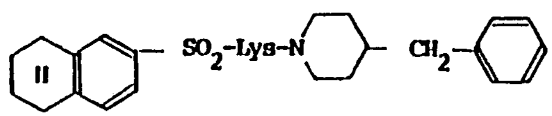 | MS:<br>M/e 497, 425, 295, 285, 195, 174, 131, 84 | NMR:<br>$CDCl_3$ , TMS<br>δ 1.16 - 2.0 (14H, m)<br>2.0 - 2.96 (7H, m)<br>3.40 - 3.72 (1H, broad)<br>3.80 - 4.36 (4H, m)<br>6.88 - 7.84 (8H,m) |
| 92 | 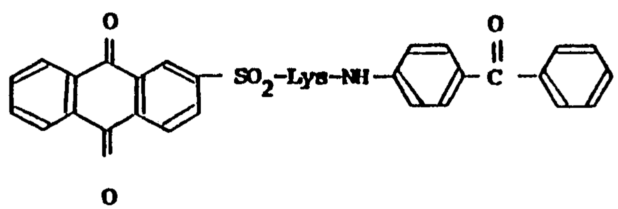 | MS:<br>M/e 446, 417, 413, 389, 341, 306, 287, 240, 223, 208, 197, 120 | IR:<br>1670, 1640, 1580, 1520, 1320, 1280, 1175, 1145 |

Table I (List of Compounds of Present Invention) (Continued)

| Compound No. | Compound | Physical Properties | |
|---|---|---|---|
| 93 | 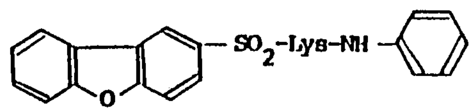 | MS:<br>M/e 358, 331, 314, 247, 231, 215, 202, 200, 183, 168, 93, 83 | NMR:<br>$(CD_3)_2SO$, TMS<br>δ 0.96 - 1.84 (6H, m)<br>2.10 - 3.08 (3H, m)<br>6.68 - 8.64 (12H, m) |
| 94 | 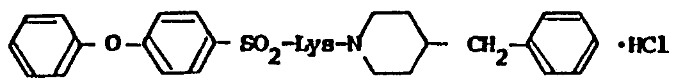 | MS:<br>M/e 535, 517, 463, 360, 333, 285, 233, 174 | NMR:<br>$CDCl_3$ , TMS<br>δ 0.90 - 1.80 (10H, broad)<br>2.40 - 3.0 (2H, m)<br>3.0 - 4.40 (6H, m)<br>6.60 - 7.92 (14H, m) |
| 95 | 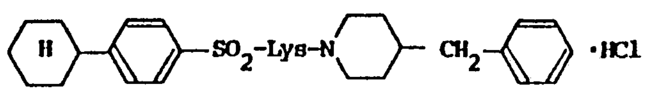 | MS:<br>M/e 525, 507, 453, 323, 285, 202, 174, 159 | NMR:<br>$CDCl_3-(CD_3)_2SO$, TMS<br>δ 0.84 - 2.04 (20H, m)<br>2.25 - 2.80 (4H, m)<br>3.24 - 4.36 (4H, m)<br>6.88 - 8.64 (9H, m) |

Table I (List of Compounds of Present Invention) (Continued)

| Compound No. | Compound | Physical Properties | |
|---|---|---|---|
| 96 | 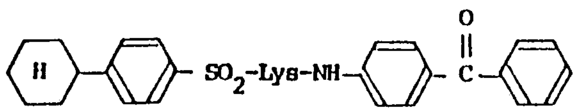 | MS:<br>M/e 382, 306, 291, 239, 223, 208, 197, 192, 183, 160, 149, 136, 80 | NMR:<br>$CD_3OD$, TMS<br>δ 0.84 - 1.88 (20H, m)<br>2.12 - 2.44 (1H, m)<br>2.64 - 3.10 (2H, m)<br>3.76 - 4.10 (1H, m)<br>7.06 - 8.28 (13H, m) |
| 97 | 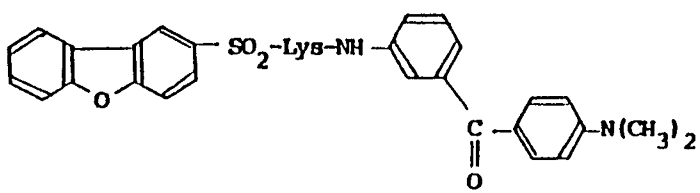 | MS:<br>M/e 398, 366, 349, 266, 247, 240, 200, 168, 148, 139 | NMR:<br>$CDCl_3$ , TMS<br>δ 1.08 - 1.88 (6H, broad)<br>2.59 (2H, broad)<br>3.06 (6H, s)<br>3.24 - 3.68 (3H, broad)<br>4.04 (1H, m)<br>6.52 - 8.12 (14H, m)<br>8.56 (1H, s) |
| 98 | 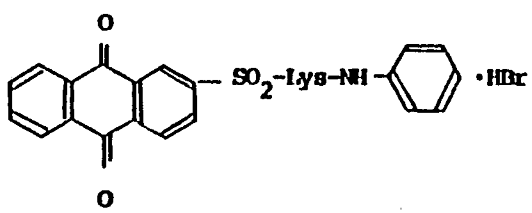 | MS:<br>M/e 446, 287, 240, 208, 119, 93, 84, 80 | |

Table I (List of Compounds of Present Invention) (Continued)

| Compound No. | Compound | Physical Properties | |
|---|---|---|---|
| 99. | 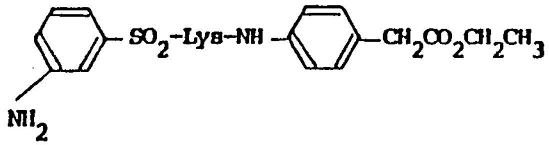 | MS:<br>M/e 309, 288, 270, 215<br>201, 179, 106, 84 | |
| 100 | 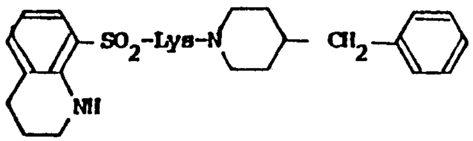 | MS:<br>M/e 498, 323, 304, 285,<br>175, 132, 84 | NMR:<br>$CDCl_3$ , TMS<br>δ 0.88 - 2.00 (13H, m)<br>2.02 - 2.88 (10H, m)<br>3.16 - 3.58 (4H, m)<br>3.68 - 3.96 (1H, m)<br>4.24 (1H, t)<br>5.84 (1H, s)<br>6.48 (1H, q)<br>6.80 - 7.48 (8H, m) |
| 101 | 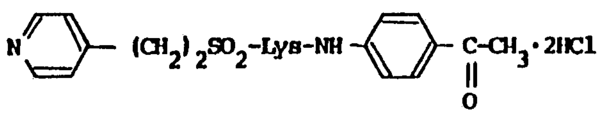 | IR:<br>3440, 1680, 1600 | NMR (free)<br>$CD_3OD$, TMS<br>δ 1.10 - 2.00 (6H, m)<br>2.55 (3H, s)<br>2.90 - 3.50 (2H, m)<br>3.30 (4H, s)<br>7.00 - 8.40 (8H, m) |
| 102 | 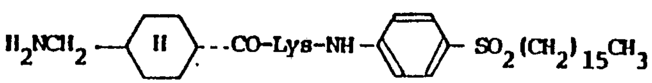 | IR:<br>3440, 1650, 1150 | |

Table I (List of Compounds of Present Invention) (Continued)

| Compound No. | Compound | Physical Properties |
|---|---|---|
| 103 | 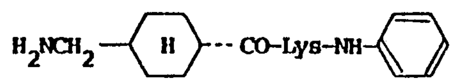 | NMR:<br>$CD_3OD$, TMS<br>δ 0.8 - 2.0 (17H, m)<br>2.2 - 2.3 (1H, broad)<br>2.5 - 2.6 (2H, m)<br>2.7 - 2.8 (2H, m)<br>2.85 - 3.2 (4H, broad)<br>4.4 - 4.12 (1H, m)<br>7.04 - 7.92 (5H, m) |
| 104 | 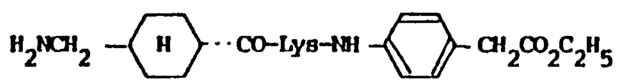 | NMR:<br>$CH_3OD$, TMS<br>δ 0.8 - 2.0 (24H, m)<br>2.20 (2H, s)<br>2.44 (2H, m)<br>2.62 (2H, m)<br>2.92 - 3.0 (2H, m)<br>3.04 - 3.12 (2H, m)<br>4.04 - 4.28 (5H, m)<br>4.30 - 4.48 (1H, m)<br>7.20 - 7.60 (4H, m) |
| 105 | 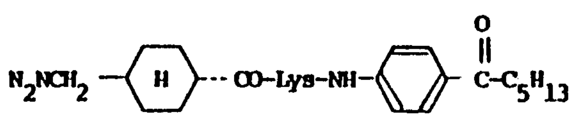 | IR:<br>1640, 1600 |

Table I (List of Compounds of Present Invention) (Continued)

| Compound No. | Compound | Physical Properties | |
|---|---|---|---|
| 106 | 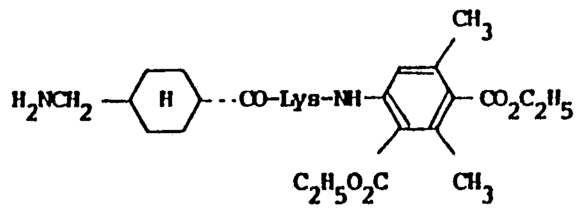 | IR: 532, 503, 487, 328, 265, 220, 191, 128, 84 | |
| 107. | 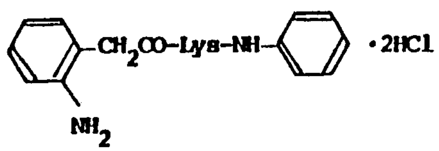 | MS: M/e 354, 336, 261, 230, 222, 221, 203, 128, 93, 84 | |
| 108 | 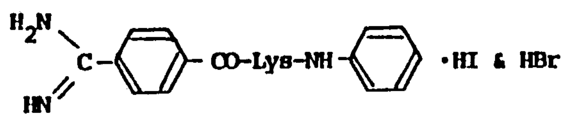 | IR: 3600 - 2400, 1680, 1600, 1520, 1490, 1445, 1300 | NMR: $CD_3OD$, TMS δ 1.40 - 1.70 (6H, broad, s) 3.16 (2H, s) 7.0 - 8.08 (9H, m) |
| 109 | 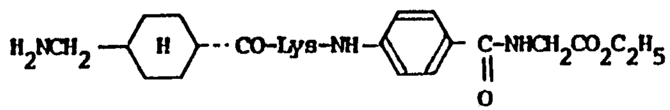 | MS: M/e 443, 250, 191, 177, 177, 136, 128, 120, 83 | |

Table I (List of Compounds of Present Invention) (Continued)

| Compound No. | Compound | Physical Properties |
|---|---|---|
| 110 | 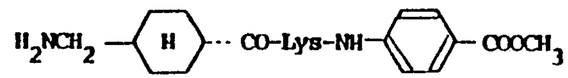 | IR: 1640, 1590, 1700 |
| 111 | 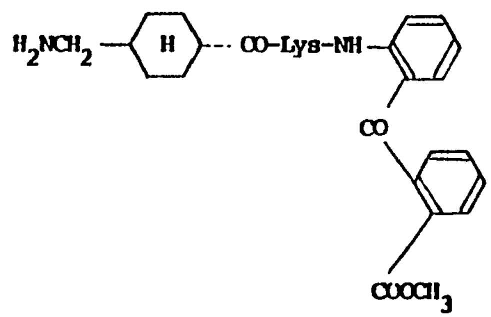 | IR: 1640, 1590, 1700, 1690 |
| 112 | 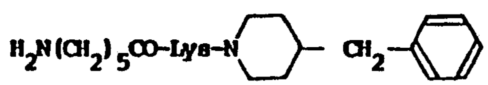 | IR: 1640, 1600 |

Table I (List of Compounds of Present Invention) (Continued)

| Compound No. | Compound | Physical Properties |
|---|---|---|
| 113 | 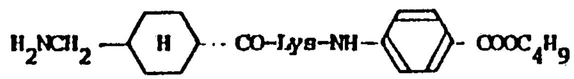 | IR: 1640, 1600, 1720 |
| 114 | 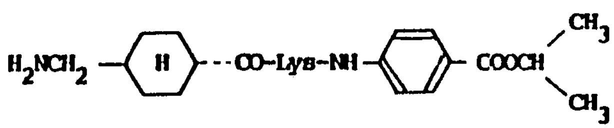 | IR: 1640, 1600, 1710 |
| 115 | 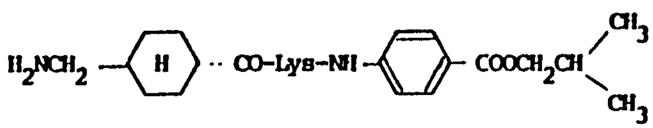 | IR: 1640, 1600, 1720 |
| 116 | 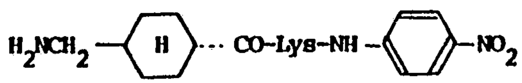 | IR: 1640, 1600, 1480, 1340 |
| 117 | 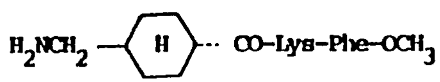 | IR: 1740, 1640, 1600 |

Table I (List of Compounds of Present Invention) (Continued)

| Compound No. | Compound | Physical Properties | |
|---|---|---|---|
| 118 | 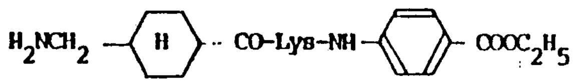 | IR:<br>1640, 1600, 1490, 1720 | |
| 119 | 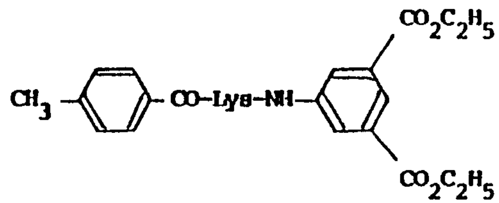 | MS:<br>M/e 483, 465, 346, 237, 246 | NMR:<br>$CDCl_3$, TMS<br>δ 1.33 (6H, t)<br>1.2 - 2.2 (6H, broad)<br>2.3 (3H, s)<br>2.6 - 3.2 (3H, broad)<br>4.35 (4H, q)<br>4.6 - 5.4 (4H, broad)<br>7.1 - 7.9 (4H, m)<br>8.3 (1H, s)<br>8.5 (2H, s) |
| 120 | 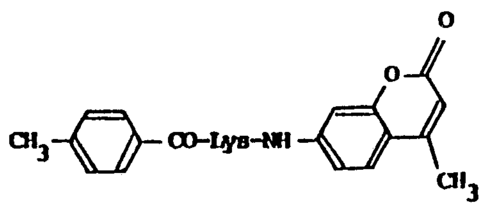 | NMR:<br>$CDCl_3$, TMS<br>δ 1.4 - 2.0 (6H, broad)<br>2.38 (6H, s)<br>2.2 - 3.0 (3H, broad)<br>4.7 - 5.2 (4H, broad)<br>7.0 - 8.0 (8H, m) | |

Table I (List of Compounds of Present Invention) (Continued)

| Compound No. | Compound | Physical Properties | |
|---|---|---|---|
| 121 | 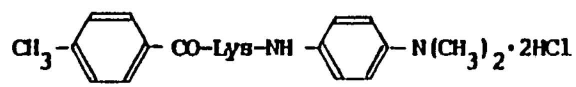 | MS:<br>M/e 382, 364, 346, 263,<br>246, 136, 119 | |
| 122 | 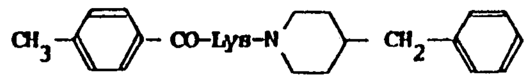 | MS:<br>M/e 421, 246, 219, 176<br>119, 84 | NMR:<br>$CDCl_3$, TMS<br>δ 0.95 - 1.95 (11H, broad)<br>2.38 (3H, s)<br>2.20 - 3.10 (7H, m)<br>3.85 - 5.20 (2H, m)<br>7.00 - 7.80 (9H, m) |
| 123 | 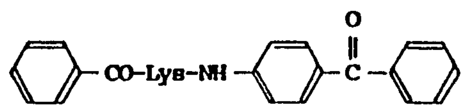 | NMR:<br>$CDCl_3$, TMS<br>δ 1.30 - 2.20 (6H, broad)<br>2.50 - 2.70 (2H, m)<br>3.20 - 3.30 (1H, broad)<br>4.10 - 5.10 (3H, m)<br>6.50 - 7.98 (14H, m) | IR:<br>3400, 1660, 1600 |
| 124 | 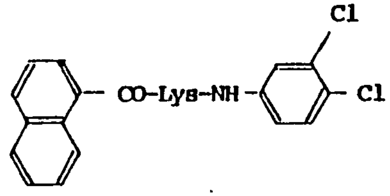 | NMR:<br>$CDCl_3$, TMS<br>δ 1.20 - 2.10 (6H, broad)<br>2.36 - 2.72 (2H, m)<br>4.96 - 5.24 (1H, m)<br>6.81 - 8.40 (10H, m) | |

Table J (List of Compounds of Present Invention) (Continued)

| Compound No. | Compound | Physical Properties | |
|---|---|---|---|
| 125 | 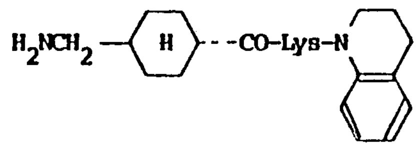 | IR:<br>1640, 1600, 1510,<br>1450, 1400 - 1490 | |
| 126 | 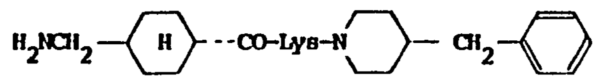 | IR:<br>1640, 1630, 1600,<br>1510, 1490, 1450 | |
| 127 | 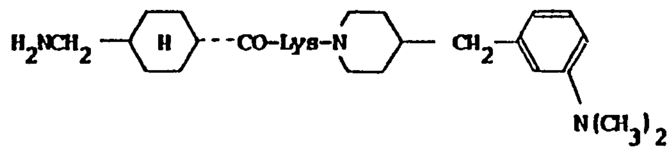 | MS:<br>M/e 485, 357, 351<br>268, 240, 219,<br>134, 84 | NMR:<br>$CD_3OD$, TMS<br>δ 0.80 - 2.00 (15H, m)<br>2.20 - 3.20 (11H, m)<br>2.88 (6H, s)<br>3.30 - 4.50 (5H, m)<br>6.70 - 7.40 (4H, m) |
| 128 | 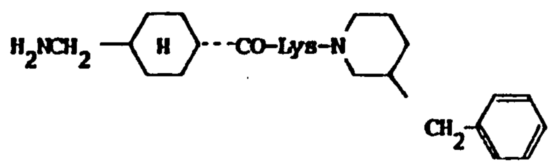 | NMR:<br>$CDCl_3$, TMS<br>δ 0.85 - 2.05 (15H, m)<br>2.20 - 2.75 (11H, m)<br>3.30 - 3.80 (4H, m)<br>4.20 (1H, m)<br>7.00 - 7.50 (5H, m) | |

Table I (List of Compounds of Present Invention) (Continued)

| Compound No. | Compound | Physical Properties | |
|---|---|---|---|
| 129 | 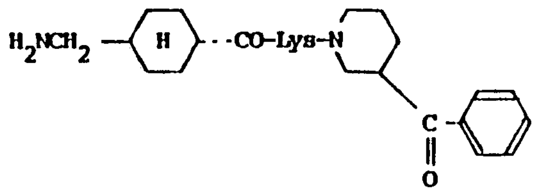 | MS:<br>M/e 326, 223, 188 | IR:<br>3375, 2900, 1670,<br>1600, 1440, 1250, 1205 |
| 130 | 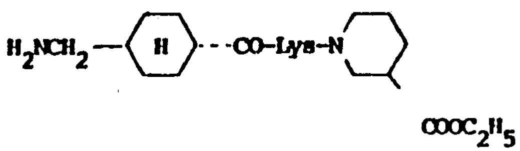 | IR:<br>1680 - 1690, 1640, 1430 | |
| 131 | 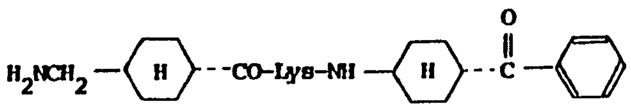 | MS:<br>M/e 470, 441, 412, 313,<br>267, 241, 204, 187,<br>157, 105, 84 | IR:<br>3270, 2920, 1680, 1660,<br>1640, 1630, 1550 |
| 132 | 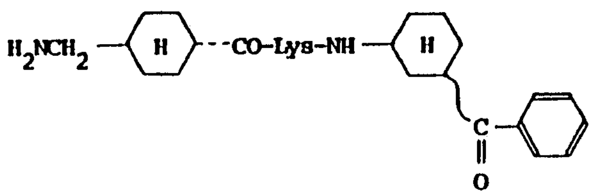 | MS:<br>M/e 470, 441, 412, 313,<br>267, 241, 204, 187,<br>157, 105, 84 | IR:<br>3275, 2925, 1670, 1660,<br>1630, 1550 |
| 133 | 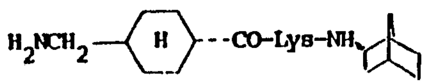 | IR:<br>1640, 1510, 1450 | |

Table I (List of Compounds of Present Invention) (Continued)

| Compound No. | Compound | Physical Properties |
|---|---|---|
| 134 | 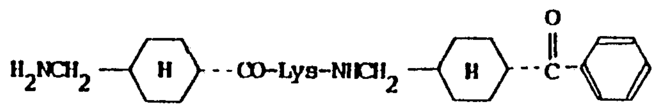 | MS:<br>M/e 484, 469, 467, 455, 426, 327, 267, 218, 110, 105, 84 |
| 135 | 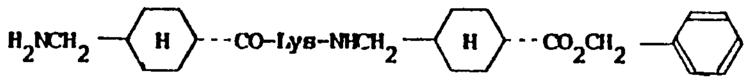 | MS:<br>M/e 514, 358, 267, 191, 128, 84 |
| 136 | 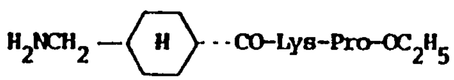 | IR:<br>1740, 1640, 1510, 1450 |
| 137 | 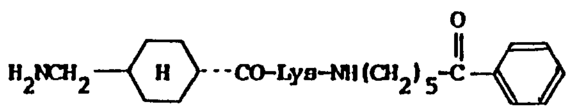 | NMR:<br>$CD_3OD$, TMS<br>δ 0.84 - 2.02 (22H, m)<br>2.12 - 2.36 (2H, m)<br>2.48 (2H, d)<br>2.64 (2H, t)<br>4.60 - 4.92 (1H, m)<br>7.08 - 8.0 (5H, m)<br>2.8 - 3.76 is not known because of overlapping with the solvent. |

Table I (List of Compounds of Present Invention) (Continued)

| Compound No. | Compound | Physical Properties | |
|---|---|---|---|
| 138 | 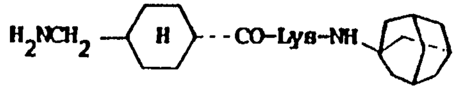 | IR: 1640, 1510, 1450 | |
| 139 | 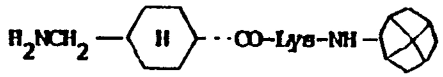 | IR: 1640, 1510, 1450 | |
| 140 | 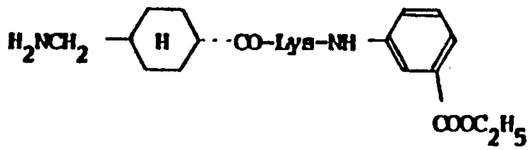 | IR: 1720 - 1730, 1640, 1600, 1490 | |
| 141 | 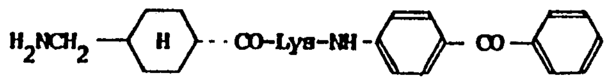 | IR: 1690, 1680, 1640, 1600, 1490 | NMR: $CD_3OD$, TMS<br>δ 0.8 - 2.0 (15H, m)<br>2.1 - 2.4 (1H, m)<br>2.65 (2H, d)<br>2.80 (2H, t)<br>4.35 - 4.55 (1H, m)<br>7.75 - 7.85 (9H, m) |
| 142 | 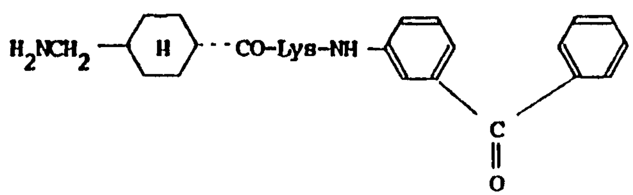 | MS: M/e 308, 279, 267, 140, 128, 84 | IR: 3350, 1630 |

Table I (List of Compounds of Present Invention) (Continued)

| Compound No. | Compound | Physical Properties | |
|---|---|---|---|
| 143 | 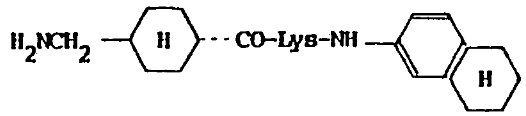 | IR:<br>1690, 1640, 1510, 1450 | |
| 144 | 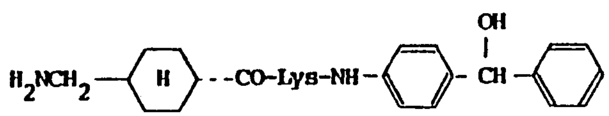 | IR:<br>3300, 2810, 1610, 1540,<br>1390 | NMR:<br>$CD_3OD$, TMS<br>δ 0.8 - 2.0 (15H, m)<br>2.08 - 2.4 (1H, m)<br>2.50 (2H, d)<br>2.64 (2H, t)<br>4.12 - 4.48 (1H, m)<br>5.72 (1H, s)<br>7.16 - 7.92 (9H, m) |
| 145 | 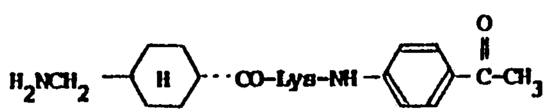 | MS:<br>M/e 402, 267, 251, 238,<br>135, 120, 110 | IR:<br>3280, 2920, 1680, 1660,<br>1600, 1540, 1280, 860 |

Table I (List of Compounds of Present Invention) (Continued)

| Compound No. | Compound | Physical Properties | |
|---|---|---|---|
| 146 | 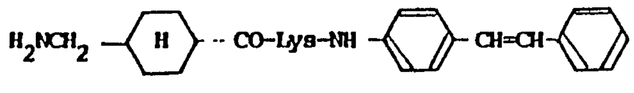 | MS: M/e 462, 267, 195, 140, 128, 84 | IR: 3360, 1650, 1510 |
| 147 | 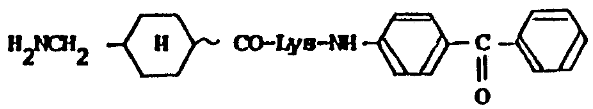 (cis rich) | IR: 1690, 1640, 1600, 1490 | |
| 148 | 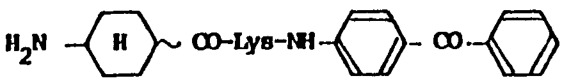 | IR: 1690, 1640, 1600, 1510, 1490, 1450 | |
| 149 | 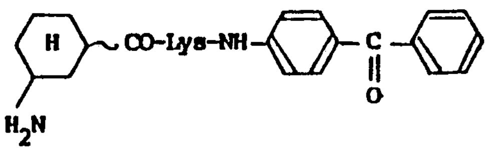 | IR: 1690, 1640, 1600, 1490 | |
| 150 | 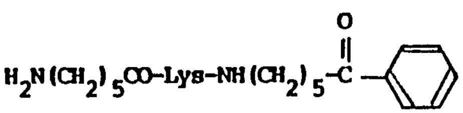 | MS: M/e 433, 415, 375, 329, 286, 243, 106, 84 | IR: 2845, 1670, 1650, 1610, 1540, 1390, 1010 |

Table I (List of Compounds of Present Invention) (Continued)

| Compound No. | Compound | Physical Properties | |
|---|---|---|---|
| 151 | 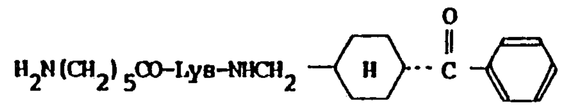 | MS:<br>M/e 458, 442, 429, 400,<br>371, 327, 218, 131,<br>105, 84 | NMR:<br>$CDCl_3$ , TMS<br>δ 0.70 - 3.50 (28H, m)<br>4.40 (2H, broad)<br>7.34 - 7.60 (3H, m)<br>7.84 - 8.00 (2H, d) |
| 152 | 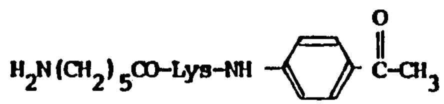 | IR:<br>3250, 2900, 1642, 1600,<br>1540, 1310, 1262, 1185,<br>858 | NMR:<br>$CD_3OD-CDCl_3$ , TMS<br>δ 0.70 - 2.78 (14H, m)<br>2.58 (3H, s)<br>3.05 - 3.60 (5H, m)<br>4.50 (2H, broad)<br>7.50 - 8.00 (5H, m) |
| 153 | 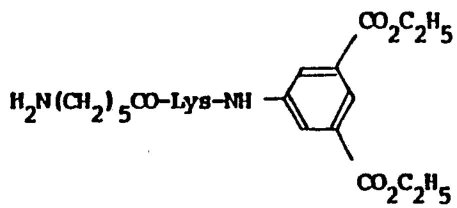 | MS:<br>M/e 265, 237 | IR:<br>3350, 1690, 1625, 1550,<br>1400, 1325, 1240 |
| 154 | 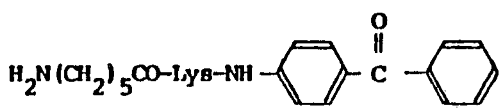 | MS:<br>M/e 362, 307, 195 | IR:<br>3350, 2900, 2830, 1630,<br>1585, 1520, 1305, 1275 |

Table I (List of Compounds of Present Invention) (Continued)

| Compound No. | Compound | Physical Properties | |
|---|---|---|---|
| 155 | 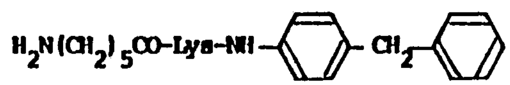 | MS:<br>M/e 241, 183, 292 | IR:<br>3320, 1640, 1620, 1550, 1400, 1300 |
| 156 | 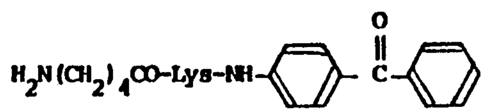 | MS:<br>M/e 306, 197 | IR:<br>3200, 2980, 2870, 2800, 1620, 1580, 1510, 1430, 1395, 1300, 1265, 1240 |
| 157 | 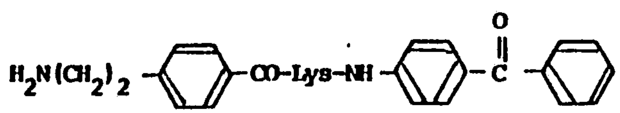 | MS:<br>M/e 306, 246, 197 | IR:<br>3350, 1620, 1560, 1480, 1400, 1320 |
| 158 | 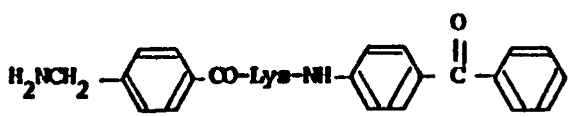 | NMR:<br>$CD_3OD$, TMS<br>δ 1.24 - 1.62 (6H, m)<br>2.48 - 2.70 (2H, m)<br>3.82 (2H, s)<br>4.36 - 4.52 (1H, m)<br>7.22 - 7.96 (13H, m) | |

Table I (List of Compounds of Present Invention) (Continued)

| Compound No. | Compound | Physical Properties |
| --- | --- | --- |
| 159 | 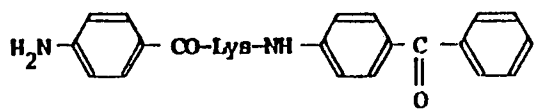 | IR: 1690, 1650, 1600, 1500 |
| 160 | 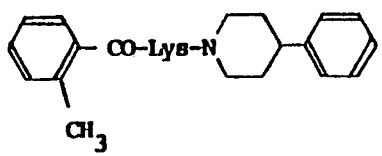 | MS: M/e 407, 378; 349, 336 271 |

The lysine derivatives according to the present invention can be synthesized by various combinations of the so-called peptide synthesis methods. The synthesis routes can be typically divided into the following two routes.

The terms "N terminal" and "C terminal" of lysine used herein means as follows.

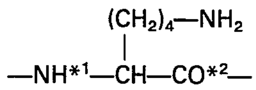

*1: N terminal
*2: C terminal

A) The N terminal group of lysine is first introduced into the starting commercially available $N^6$-benzyloxycarbonyl-L-lysine (i.e.,

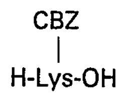

wherein —CBZ= —$COOCH_2$—ф) and the C terminal group of lysine is then introduced thereinto, followed by removing the protective group CBZ.

B) The C terminal group of lysine is first introduced into the starting commercially available $N^2$-t-butoxycarbonyl-$N^6$-benzyloxycarbonyl-L-lysine (i.e.,

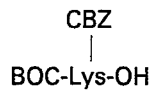

wherein BOC= —COO—$CH_3)_3$), the BOC group is then selectively removed therefrom in a known manner, and the N terminal group of lysine is further introduced, followed by removing the CBZ group.

Furthermore, in the practice of the introduction of the N terminal group and the C terminal group, the following methods can be utilized:

(a) The introduction of the N terminal group can be introduced by using aromatic sulfonyl chlorides (i.e., $ArSO_2Cl$) or aromatic carbonyl chlorides (i.e., ArCOCl).

(b) The introduction of the C terminal group can be introduced by the following known methods.

(i) Mixed acid anhydride method [Ann, Chem., *572,* 190 (1951)].

(ii) Acid chloride method Biochemistry, *4,* 2219 (1960)].

(iii) Phosphazo method [Chem. Ber., *93,* 2387 (1960)].

(iv) N,N'-dicyclohexylcarbodiimide method [J. Am. Chem. Soc., *77,* 1067 (1955)].

(v) Active ester method using, for example, N-hydroxysuccinimide [J. Am. Chem. Soc., *85,* 3039 (1963)].

It should be noted, however, that the desired synthesis methods must be selected by appropriately combining the above-mentioned methods. Typical routes for synthesizing the lysine derivatives are exemplified as follows. In the following routes, the amine portion represented by

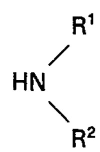

may be substituted with

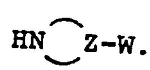

Route (1)

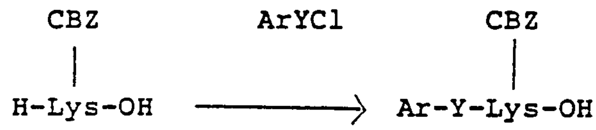


(1) (2)

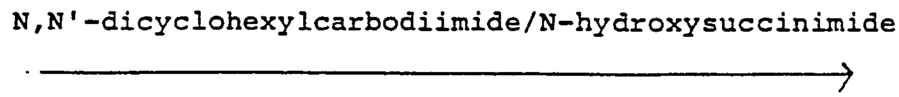


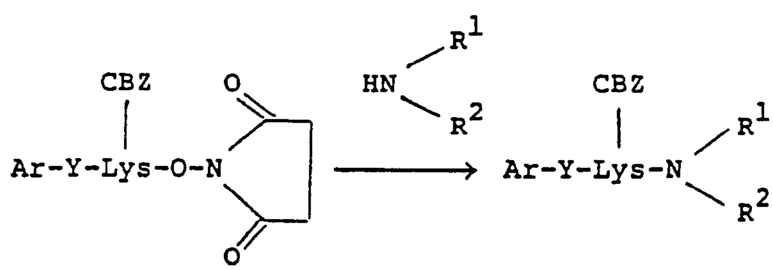

(3) (4)

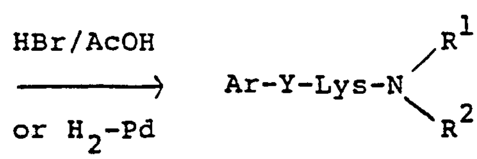


(5)

The synthesis of the compound (2) from the compound (1) can be carried out by using the so-called Schotten-Baumann reaction. That is, the starting compound (1) is dissolved or suspended in a suitable solvent system (e.g., ethyl ether-water, toluene-water, 1,4-dioxane-water, acetone-water and a suitable base (e.g., NaOH, $NaHCO_3$) is added in an amount of, for example, 1 to 5 equivalent, preferably 2 to 3 equivalent, to the compound (1). To the resultant mixture, an aromatic sulfonyl or an aromatic carbonyl chloride (i.e., $ArSO_2Cl$ or ArCOCl) is added alone or as a solution in an organic solvent used in the reaction medium. The addition may be carried out all at once or in several portions. The reaction is generally carried out at a temperature of −10°C to 30°C, preferably 5°C to 10°C for 1 to 50 hours, preferably 5 to 20 hours. The compound (2) can be recovered from the reaction mixture in any conventional manner.

The synthesis of the compound (3) from the compound (2) can be carried out by the method (b)-(v) set forth above.

The compound (4) can be prepared from the compound (3) as follows. That is, the compound (3) is dissolved in a suitable organic solvent (e.g., ethers, hydrocarbons, halogenated hydrocarbons, N,N'-dialkylformamides, nitriles) and a 1 to 3 equivalent amount of

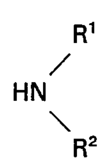

is added thereto. The reaction is generally carried out at at temperature of −10°C to 30°C, preferably 0°C to

20°C for 1 to 50 hours, preferably 5 to 20 hours. After completing the reaction, the compound (4) can be recovered in any conventional manner.

The synthesis of the compound (5) from the compound (4) can be carried out by the so-called HBr/AcOH method [see J. Am. Chem. Soc., *81,* 5688 (1959)] or the so-called $H_2$-Pd catalytic hydrogenation method [see Chem. Ber., *65,* 1192 (1932)].

Route ②

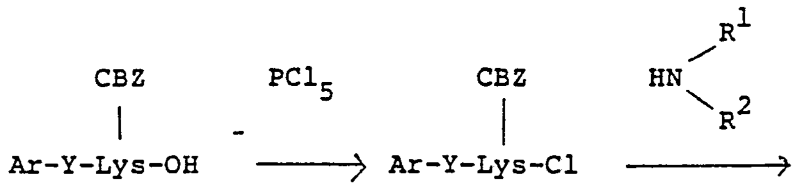


(1) (2)

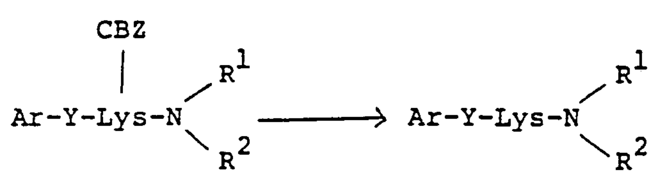

(3) (4)

The compound (1) is dissolved in a suitable dried solvent (e.g., ethers, halogenated hydrocarbons) and, while the reaction temperature is maintained at −10°C to 30°C, preferably 0°C to 5°C, a 1.0 to 5.0 equivalent, preferably 1.0 to 1.5 equivalent, amount of phosphorus pentachloride is added all at once or over a period of 10 minutes to 1 hour, preferably 10 to 20 minutes, with stirring. After the addition, the reaction mixture is further stirred for 30 minutes to 1 hour while maintaining the above-mentioned temperature range. Thereafter, the reaction mixture is allowed to stand with stirring at room temperature for 10 minutes to 2 hours, preferably for 10 minutes to 1 hour. The solvents and the other volatile substances are distilled off in vacuo at temperature of 10°C to 70°C, preferably 30°C to 50°C. Thus, the compound (2) can be obtained.

Since the compound (2) is unstable, the synthesis of the compound (3) from the compound (2) is preferably carried out immediately. That is, the compound (2) is dissolved in a suitable dried solvent (e.g., ethers, halogenated hydrocarbons, hydrocarbons) and a 1 to 3 equivalent amount of

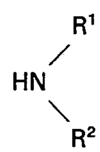

is added thereto. In this case, tertially organic amines such as triethylamine may be used. The reaction is generally carried out at a temperature of 0°C to 50°C preferably 10°C to 20°C for 1 to 50 hours, preferably 5 to 20 hours. After completing the reaction, the compound (3) can be recovered in any conventional post-treatment method.

The synthesis of the compound (4) from the compound (3) can be carried out in the same manner as in the above-mentioned route ①.

Route ③

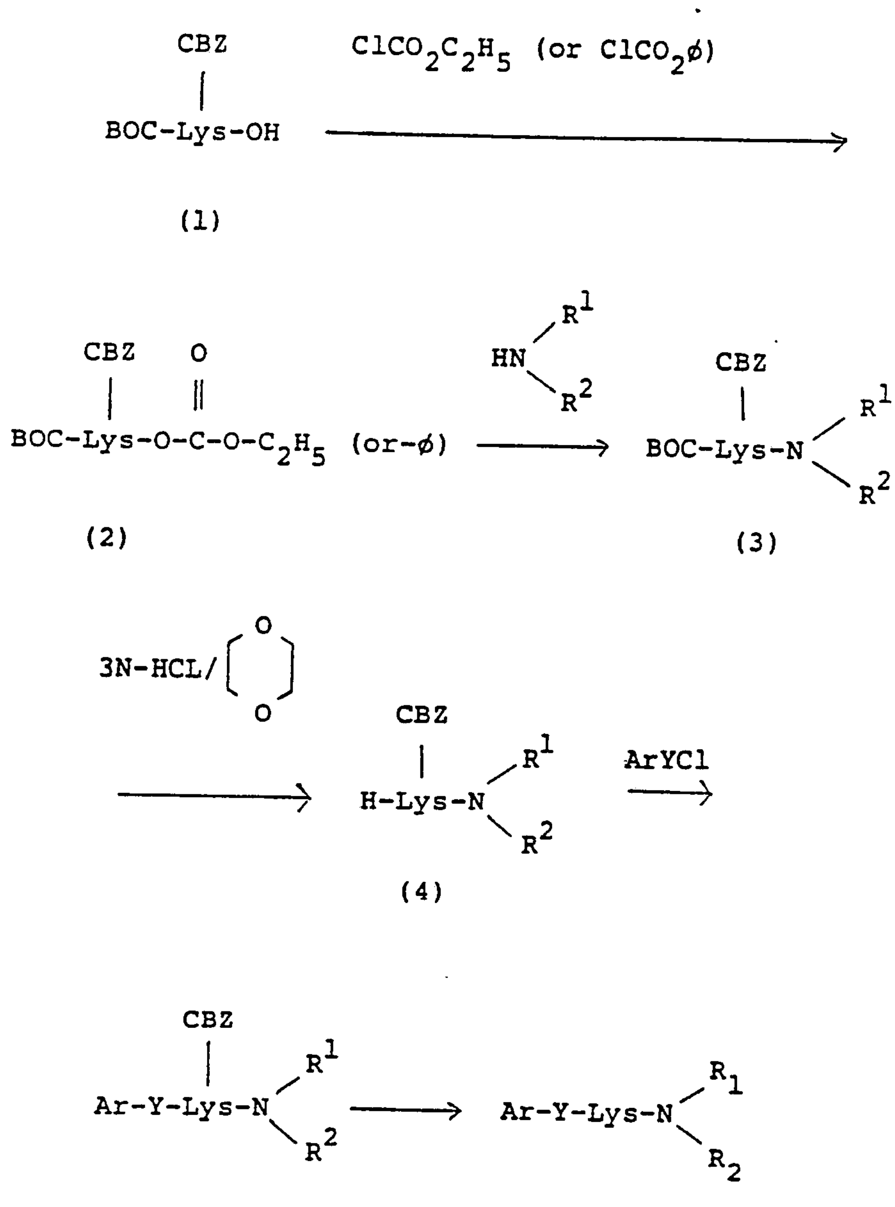


(1) (2) (3) (4) (5) (6)

In the route ③, the starting commercially available compound (1) is first dissolved in a suitable dried solvent (e.g., ethyl acetate, 1,4-dioxane, tetrahydrofuran) and a 1 t 5 equivalent amount, preferably a 1 to 2 equivalent amount, of a suitable tertially organic amine (e.g., triethylamine) is added thereto in an amount of 1 to 5 equivalent, preferably 1 to 2 equivalent, to the compound (1). The resultant solution is cooled to a temperature of −20°C to 10°C, preferably −15°C to 0°C. Then, a 1 to 3 equivalent, preferably 1 to 1.5 equivalent amount of ethyl chlorocarbonate (or phenyl chlorocarbonate) is added to the cooled solution and the reaction is carried out for 5 minutes to one hour with stirring. After completing the reaction, a solution containing the compound (2) can be obtained in any conventional post-treatment method.

To the solution obtained above, a 1 to 3 equivalent of

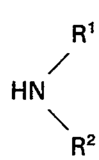

is added at a temperature of 15°C to 0°C. After the addition, the mixture is allowed to react at the same temperature for 10 minutes to 5 hours, and at a temperature of 5°C to 30°C, preferably 10°C to 20°C for 10 to 50 hours. After completing the reaction, the compound (3) can be recovered in any conventional post-treatment method.

The synthesis of the compound (4) from the compound (3) can be carried out by a known method as disclosed in, for example, Proc. Natl. Acad. Sci., *58,* 1806 (1967).

The synthesis of the compound (5) from the compound (4) can be carried out either by using the so-called Schotten-Baumann reaction set forth in the above-mentioned route ① or by using a suitable organic solvent (e.g., ethers, N,N-dialkylformamide, N,N-dialkylacetamide, halogenated hydrocarbons) in combination with a suitable tertially organic base (e.g., trialkylamines, dialkylanilines, pyridine).

The synthesis of the compound (6) from the compound (1) can be carried out in the same manner as in the above-mentioned route ①.

Furthermore, in the case where the amino group is contained as a terminal group of the N terminal of lysine, the lysine derivative according to the present invention can be similarly prepared in the following routes ④ and ⑤.

The L-lysine derivatives obtained above can be converted to the pharmaceutically acceptable salts thereof in any conventional manner.

Route ④

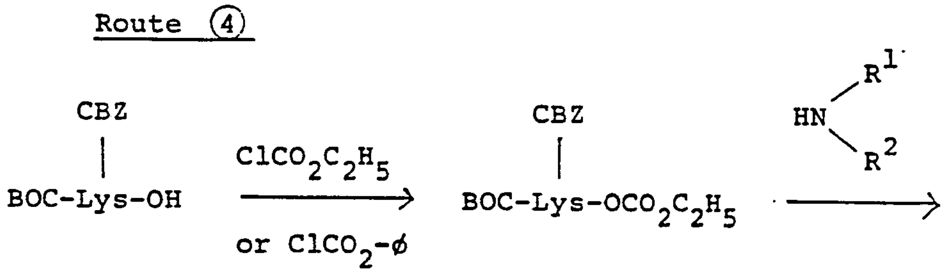


(1) (2)

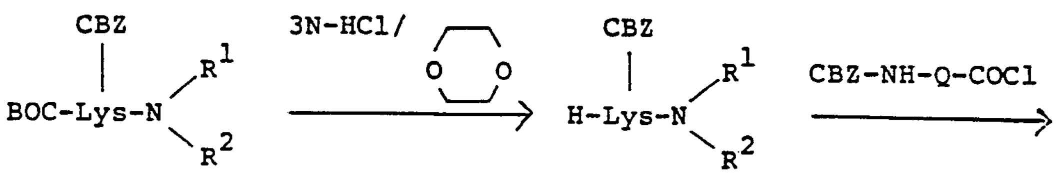


(3) (4)

wherein Q is a residue of the group A define above from which a group $NH_2$ is removed.

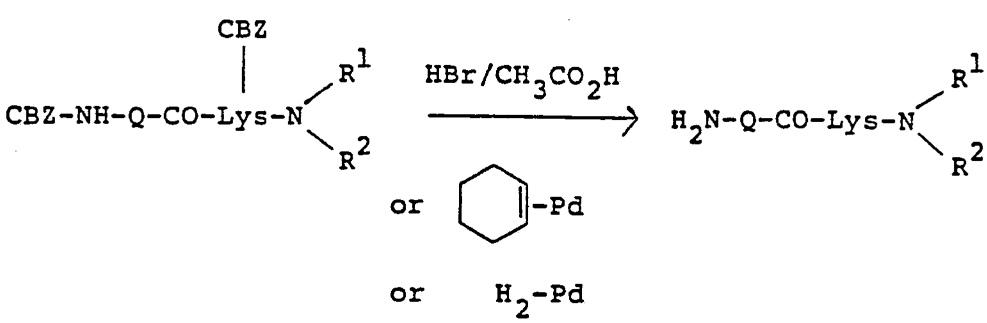


(5) (6)

Route (5)

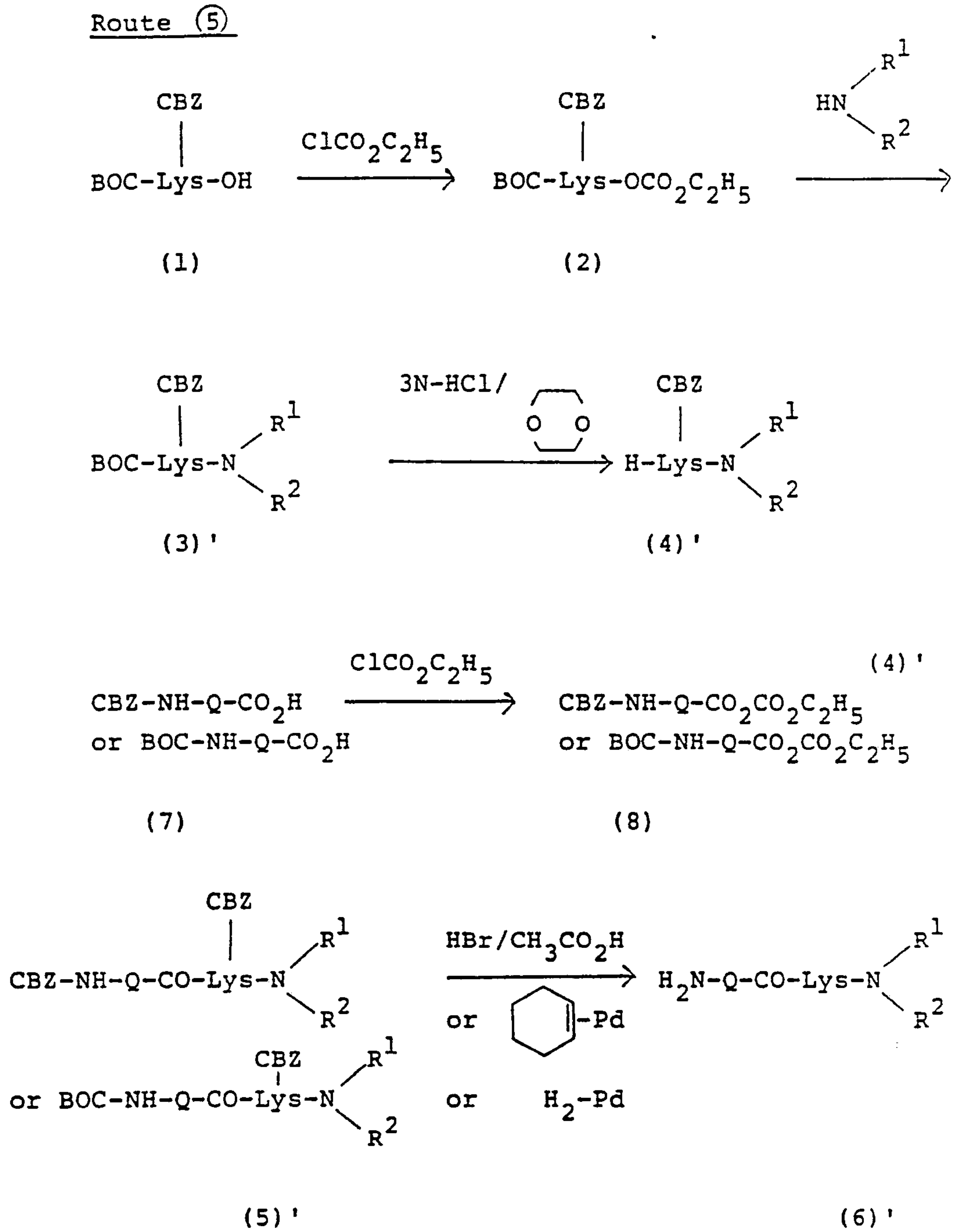


(1) (2) (3)' (4)' (7) (8) (5)' (6)'

The L-lysine derivatives or the pharmaceutically acceptable salts thereof according to the present invention, which are an effective component of the proteinase inhibitor of the present invention have remarkable inhibition activities against proteinases such as plasmin, kallikrein, trypsin, and urokinase as shown in the below-mentioned test results shown in Tables IV and V. It has not been reported that the low-molecular weight compounds exhibiting no substantial inhibition activities against thrombin exhibit the above-mentioned unique enzyme inhibition pattern. Furthermore, ε-aminocaproic acid, tranexamic acid and other compounds, which are heretofore widely uses as plasmin inhibitors, have an activity capable of selectively inhibiting the fibrin dissolving action of plasmin and, therefore, are used as useful hemostatics. This pharmacological action is believed to be effected by the fact that these compounds are bonded to the so-called lysine binding sites (i.e., LBS) of plasminogen and plasmin, whereby the binding of fibrin to the plasminogen and plasmin is prevented as reported in, for example, Chem. Rev., *81,* 431 (1981), Biochem. J., *163,* 389 (1977), and Eur. J. Biochem., *84,* 573 (1978). These compounds have no substantial activities to prevent the decomposition of synthetic substrates (e.g., S—2251 available from Kabi Co., Ltd.) and

fibrinogen caused by plasmin. This means that, although various substrates (e.g., fibrinogen), other than fibrin, are present in the human organisms for plasmin the above-mentioned compounds are not effective for preventing the decomposition of these substrates.

Contrary to the above, the proteinase inhibitors according to the present invention have remarkable inhibition activities against the decomposition of the synthetic substrates and fibrinogen as well as the decomposition of fibrin by plasmin and, therefore, are novel antiplasmins suitable for use as a hemostatic agent against hemorrhagic disorders and inflammatory disorders.

On the other hand, known compound Nos. 4, 5, 7, and 8 are listed in Table II having a structure similar to those of the present compounds has only a very low inhibition activity against the action of plasmin as shown in Table III. It is clear from the comparison of the results in Tables III and IV that the inhibition activities of the present compounds shown in Table IV are far superior to that of said compounds.

Furthermore, as shown in Table V, some L-lysine derivatives according to the present invention have inhibition activities against urokinase, which is a plasminogen activating enzyme. This means that the present L-lysine derivatives provide favorable results as a hemostatic agent. In addition, some L-lysine derivatives according to the present invention exhibit inhibition activities against kallikrein and trypsin. This means that these inhibition activities can provide, together with the antiplasmin activity, a strong antiinflammatory agent.

When the L-lysine derivatives or the pharmaceutically acceptable salts thereof are used as a medicine, there are no critical limitations to the administration methods. The present proteinase inhibitor can be formulated by any conventionel method. For example, the present proteinase inhibitor may be applied in any conventional manner including intravenous injection, intramuscular injection, subcutaneous injection, intravenous drip, and oral administration. Although there are no critical limitations to the administration dosage, the suitable dosage is 100 to 1000 mg/day/person.

## Examples

The present invention will now be further illustrated by, but is by no means limited to, the following Examples illustrating the synthesis of the present compounds as well as the pharmacological test data for the evaluation thereof.

### Example 1

Synthesis of $N^2$-(p-toluenesulfonyl)-L-lysine-4-benzylpiperidinamide (i.e., Compound No. 1)

A 5 g amount of $N^6$-benzyloxycarbonyl lysine (I) was dissolved in 100 ml of 1,4-dioxane, 150 ml of water, and 4.92 g of $K_2CO_3$. A solution of 3.74 g of p-toluenesulfonyl chloride in 15 ml of 1,4-dioxane was dropwise added to the solution for 1.5 hours. The resultant mixture was allowed to stand with stirring for one night, while maintaining the temperature at 15°C. Thereafter, the 1,4-dioxane and water were distilled off in vacuo.

Water was charged to the residue and the resultant mixture was then washed with ethyl ether. The resultant two phases were separated and the aqueous phase was extracted with ethyl acetate after acidifying the aqueous phase by the addition of hydrochloric acid.

The extract was treated in a conventional manner, followed by crystallizing from ethanol-n-hexane to obtain 5.0 g of $N^2$-(p-toluenesulfonyl)-$N^6$-benzyloxycarbonyl-L-lysine (II).

A 2.2 g amount of the compound (II) and 580 mg of N-hydroxysuccinimide were dissolved in 20 ml of 1,4-dioxane. Then, 1.05 g of N,N'-dicyclohexylcarbodiimide (DCC) was added and the mixture was allowed to stand for one night at a temperature of 5°C to 10°C. Thereafter, the mixture was treated in a conventional manner to obtain 2.6 g of $N^2$-(p-toluenesulfonyl)-$N^6$-(benzyloxycarbonyl)-L-lysine N-hydroxysuccinimide ester (III).

A 1.06 g amount of the compound (III) was dissolved in 15 ml of 1,4-dioxane and 350 mg of 4-benzylpiperidine was then added. The mixture was allowed to react at a temperature of 10°C for 10 hours, while stirring. The reaction mixture was then treated in a conventional manner to obtain 820 mg of $N^2$-(p-toluene sulfonyl)-$N^6$-benzyloxycarbonyl-L-lysine 4-benzylpiperidinamide (IV).

A 1.5 ml amount of a 30% hydrogen bromide in acetic acid solution was added to 820 mg of the compound (IV). After the mixture was agitated at room temperature for 20 minutes, diethyl ether was added to precipitate the desired $N^2$-(p-toluenesulfonyl)-L-lysine 4-benzylpiperidinamide hydrobromide (V). The other was removed by decantation. After the ether washing was repeated several times, an aqueous sodium bicarbonate solution was added to the washed precipitate so that the resultant mixture became alkaline. The alkaline mixture was extracted with chloroform, followed by a conventional treatment. Thus, 650 mg of the desired compound (V) was obtained.

### Example 2

Synthesis of $N^2$-(dibenzofuran-2-sulfonyl)-L-lysine-3-benzoylanilide (i.e., Compound No. 70)

A 980 mg amount of $N^2$-(dibenzofuran-2-sulfonyl)-$N^6$-(benzyloxycarbonyl)-L-lysine (I) prepared in the same manner as in Example 1 was dissolved in 5 ml of 1,4-dioxane and 5 ml of tetrahydrofuran. Then, 800 mg of phosphorus pentachloride was dropwise added to the solution under ice cooling for 10 minutes with stirring. The stirring was continued for a further 10 minutes.

The resultant mixture was stirred at room temperature for 30 minutes and then the mixture was

distilled in vacuo at a temperature of 45°C in a water bath to remove 1,4-dioxane and other elements. Then, 10 ml of 1,4-dioxane was charged to the residue and 380 mg of 3-benzoylaniline was added thereto. The mixture was allowed to stand at room temperature for one night. The resultant mixture was then treated in a conventional manner to obtain 890 mg of $N_2$-(dibenzofuran-2-sulfonyl)-$N^6$-(benzyloxycarbonyl)-L-lysine 3-benzoylanilide (II).

A 890 mg amount of the compound (II) was treated with 2.0 ml of a 30% hydrogen bromide in acetic acid solution to obtain 130 mg of the desired $N_2$-(dibenzofuran-2-sulfonyl)-L-lysine 3-benzoylanilide.

Example 3

Synthesis of $N^2$-(coumarin-6-sulfonyl)-L-lysine-4-benzylpiperidinamide (i..e., Compound No. 80)

A 1.0 g amount of $N^2$-(t-butyloxycarbonyl)-$N^6$-(benzyloxycarbonyl)-L-lysine and 320 mg of triethylamine were dissolved in 10 ml of tetahydrofuran. While the solution was cooled in an ice-salt bath, 330 mg of ethyl chlorocarbonate was added with stirring. About 20 minutes later 460 mg of 4-benzylpiperidine was added. After stirring for 2 hours, the mixture was allowed to stand at room temperature for one night. The mixture was then treated in a conventional manner to obtain 1.1 g of $N^2$-(t-butyloxycarbonyl)-$N^6$-(benzyloxycarbonyl)-L-lysine 4-benzylpiperidinamide (I).

A 1.1 g amount of the compound (I) was dissolved in 3.5 ml of 6N-hydrogen chloride-1,4-dioxane and the mixture was stirred at room temperature for about 5 minutes. A 3.5 ml amount of 1,4-dioxane was further added and the mixture was allowed to stand at room temperature for one hour. Then, 20 ml of ethyl ether was added to settle oily $N^6$-benzyloxycarbonyl-L-lysine 4-benzylpiperidin amide hydrochloride (II). The ethyl ether was separated by decantation. After this procedure was repeated several times, an aqueous sodium bicarbonate solution was added and the compound (II) was then extracted with chloroform. The extract was dried over sodium sulfate and the chloroform was distilled off in vacuo.

A 500 mg amount of the compound (II) was dissolved in a solution of 630 mg of potassium carbonate dissolved in 6 ml of water and 20 ml of 1,4-dioxane and 280 mg of coumarin-6-sulfonyl chloride was added thereto. The mixture was treated in the same manner as in Example 1 to obtain 350 mg of $N^2$-(coumarin-6-sulfonyl)-$N^6$-(benzyloxycarbonyl)-L-lysine 4-benzylpiperidine (III).

A 260 mg amount of the compound (III) was treated with 0.5 ml of a 30% hydrobromic acid in acetic acid solution to obtain 50 mg of the desired $N^2$-(coumarin-6-sulfonyl)-L-lysine 4-benzylpiperidin amide.

Example 4

Synthesis of $N^2$-(p-toluene sulfonyl)-L-lysine p-nitroanilide hydrochloride (i.e., Compound No. 3)

A 1.4 g amount of p-nitroaniline was dissolved in 20 ml of pyridine and, while cooling in an ice-salt bath, 0.71 g of phosphorus trichloride was added thereto, followed by stirring for 15 minutes.

After the temperature of the mixture had returned to room temperature, 4.3 g of $N^2$-(p-toluenesulfonyl)-$N^6$-(benzyloxycarbonyl)-L-lysine was added thereto and the resultant mixture was stirred at a temperature of 60°C for 3 hours. The resultant mixture was then treated in a conventional manner to obtain 3.5 g of $N^2$-(p-toluenesulfonyl)-$N^6$-(benzyloxycarbonyl)-L-lysine p-nitroanilide (I).

The benzyloxycarbonyl group at $N^6$ position was removed from 1.2 g of the compound (I) in the same manner as in Example 1 to obtain 380 mg of $N^2$-(p-toluenesulfonyl)-L-lysine p-nitroanilide hydrochloride.

Example 5

Synthesis of $N^2$-(p-toluenesulfonyl)-L-lysine 4-cyanoanilide (i.e., Compound No. 26)

A 1.0 g amount of $N^2$-(p-toluenesulfonyl)-$N^6$-(benzyloxycarbonyl)-L-lysine and 270 mg of p-cyanoaniline were added to 15 ml of toluene and, while stirring, 200 mg of phosphorus trichloride was added at room temperature over 5 minutes. The resultant reaction mixture was allowed to react under reflux in an oil bath at a temperature of 120°C for 3.5 hours while stirring.

The resultant reaction mixture was then treated in a conventional manner to obtain 980 g of $N^2$-(p-toluenesulfonyl)-$N^6$-(benzyloxycarbonyl)-L-lysine 4-cyanoanilide (I). The benzyloxycarbonyl group at $N^6$ position of the compound (I) was removed from the compound (I) in the same manner as in Example 1 to obtain 510 mg of the desired $N^2$-(p-toluenesulfonyl)-L-lysine 4-cyanoanilide.

Example 6

Synthesis of $N^2$-(p-toluenesulfonyl)-L-lysine 4-nitrobenzylamide acetate (i.e., Compound No. 16)

A 4.3 g amount of $N^2$-(p-toluenesulfonyl)-$N^6$-(benzyloxycarbonyl)-L-lysine and 1.55 g of p-nitrobenzylamine were dissolved in 5 ml of N,N-dimethylformamide and 5 ml of acetonitrile and, while cooling in an ice-salt bath, 2,5 g of N,N'-dicyclohexylcarbodiimide was added thereto. The resultant mixture was allowed to react for one hour and was then allowed to stand at room temperature for one night. The reaction mixture was treated in a conventional manner to obtain 2.9 g of $N^2$-(p-toluene sulfonyl)-$N^6$-(benzyloxycarbonyl)-L-lysine 4-nitrobenzylamide acetate (I).

The benzyloxycarbonyl group at $N^6$ position was removed from 430 mg of the compound (I) in the same manner as in Example 1 to obtain 340 mg of the desired $N^2$-(p-toluenesulfonyl)-L-lysine 4-nitrobenzylamide acetate.

Example 7

Synthesis of $N^2$-(trans-4-aminomethylcyclohexylcarbonyl)-L-lysine 4-isopropyloxycarbonylanilide (i.e., Compound No. 115)

A 0.54 g amount of 4-isopropyloxycarbonylaniline was dissolved in 20 ml of N,N-dimethylformamide and the mixture was stirred under ice cooling. On the other hand, 1.14 g of $N^2$-(t-butyloxycarbonyl-$N^6$-(benzyloxycarbonyl)-L-lysine (I) was dissolved in 40 ml of dry tetrahydrofuran and, while ice cooling, 300 mg of triethylamine was added. Then, while ice-salt cooling, 330 mg of ethyl chlorocarbonate was added, followed by stirring for 15 minutes. This solution was added to the above-prepared solution and the mixture was allowed to stand at a temperature of 4°C for one night. The reaction mixture was then treated in a conventional manner to obtain 1.2 g of $N^2$-(t-butyloxycarbonyl-$N^6$-(benzyloxycarbonyl)-L-lysine 4-isopropyloxycarbonylanilide (II).

A 1.2 ml amount of a 6N-hydrogen chloride in dioxane solution was added to 360 mg of the compound (II)] while ice cooling. Ten minutes later, 1.2 ml of dioxane was added thereto and the mixture was stirred at room temperature for 30 minutes. Twenty minutes later, 50 ml of N,N-dimethylformamide was added to the resultant reaction solution while ice cooling, followed by adding 0.9 ml of triethylamine.

On the other hand, 0.2 g of trans-4-benzyloxycarbonyl aminomethylcyclohexanecarboxylic acid was dissolved in 5 ml of chloroform and 0.18 ml of thionyl chloride was added under room temperature. After the mixture was allowed to stand for 5 hours, the mixture was added to the above-prepared reaction solution and the mixture was allowed to stand at room temperature for 12 hours. The resultant reaction mixture was treated in a conventional manner to obtain 150 mg of $N^2$-(trans-4-benzyloxycarbonylamino-methylcyclohexylcarbonyl)-L-lysine 4-isopropyloxycarbonyl anilide (III].

A 84 mg amount of the compound (III) as suspended in ethanol and 10 mg of palladium black was added thereto. Thus, the reaction was carried out at room temperature for 9 hours with stirring under a hydrogen gas flow. As a result, 33 mg of the desired $N^2$-(trans-4-aminomethylcyclohexylcarbonyl)-L-lysine 4-isopropyloxycarbonyl anilide (IV) was obtained from a ether-petroleum ether solvent.

Example 8

Synthesis of $N^2$-(trans-4-aminomethylcyclohexylcarbonyl)-L-lysine 4-(ethoxycarbonylmethyl) carbamoyl anilide (i.e., Compound No. 109)

A 744 mg amount of trans-4-aminomethylcyclohexanecarboxylic acid and 574 mg of triethylamine were dissolved in 16 ml of tetrahydrofuran and, while cooling in an ice-salt bath, 282 mg of ethyl chlorocarbonate was added thereto with stirring. After stirring for 20 minutes, 1.0 g of $N^6$-benzyloxycarbonyl-L-lysine 4-(ethoxycarbonylmethyl) carbamoyl anilide hydrochloride (I) prepared in a conventional manner was added. The mixture was stirred for about 2 hours under cooling and was then allowed to stand at room temperature for one night.

Example 9

Synthesis of $N^2$-(p-toluoyl)-L-lysine 3,5-diethoxycarbonylanilide (i.e., Compound No. 119)

A 5 g amount of $N^6$-benzylcarbonyl-L-lysine (I) was dissolved in 100 ml of 1,4-dioxane, 150 ml of water, and 4.92 g of potassium carbonate. While maintaining the solution at a temperature of 10°C, a solution of 4.17 g of p-toluenecarbonyl chloride dissolved in 15 ml of 1,4-dioxane was dropwise added thereto with stirring for 2 hours. After the resultant mixture was further maintained at a temperature of 10°C for 3 hours, the mixture was allowed to stand for one night at 4°C. The 1,4-dioxane and water were distilled off and, after adding water thereto, the mixture was washed with ethyl ether. The resultant two phases were separated and, after acidifying the aqueous phase by adding hydrochloric acid, the aqueous phase was extracted with ethyl acetate. The extract was treated in a conventional manner. The product was crystallized from acetone to obtain 4.3 g of $N^2$-(p- toluoyl)-$N^6$-benzyloxycarbonyl-L-lysine (II).

A 796 amount of the compound (II) and 474 mg of 3,5-diethoxycarbonylaniline wre added to 15 ml of toluene and 200 mg of phosphorus trichloride was added thereto at room temperature for 5 minutes with stirring. The reaction mixture was refluxed upon heating with stirring for 2.5 hours. The reaction mixture was treated in a conventional manner to obtain 910 mg of $N^2$-(p-toluoyl)-$N^6$-benzyloxycarbonyl-L-lysine 3,5-diethoxycarbonylanilide (III).

A 1.5 ml amount of a 30% hydrogen bromide in acetic acid solution was added to 800 mg of the compound (III) and the mixture was stirred at room temperature for 15 minutes. Then, diethyl ether was added to precipitate the desired $N^2$-(p-toluoyl)-L-lysine 3,5-diethoxycarbonylanilide (IV) in the form of a hydrobromide salt. After the ether was removed by decantation and the ether washing was repeated several times, an aqueous sodium bicarbonate solution was added thereto and the resultant alkaline mixture was extracted with chloroform. The extract was treated in a coventional manner to obtain 230 mg of the desired compound (IV).

Example 10

Synthesis of $N^2$-(p-toluoyl)-L-lysine 4-methyl-7-coumarinyl amide (i.e., Compound No. 120)

A 774 mg amount of $N^2$-(p-toluoyl)-$N^6$-(benzyloxycarbonyl)-L-lysine (I) and 343 mg of 7-amino-4-methyl coumarin were added to 15 ml of toluene and 200 mg of phosphorus trichloride was added thereto at room temperature for 5 minutes with stirring. The reaction mixture was refluxed upon heating for 3

hours with stirring. The resultant mixture was treated in a conventional manner to obtain 765 mg of $N^2$-(p-toluoyl)-$N^6$-(benzyloxycarbonyl)-L-lysine 4-methyl-7-coumarinyl amide (II).

A 720 mg amount of the compound (II) was treated with 1.5 ml of a 30% hydrogen bromide in acetic acid solution to obtain 210 mg of the desired $N^2$-(p-toluoyl)-L-lysine 4-methyl-7-coumarinylamide.

Example 11

Synthesis of $N^2$-(1-naphthalenecarbonyl)-L-lysine 3,4-dichloroanilide (i.e., Compound No. 124)

A 500 mg amount of $N^2$-(1-naphthalene carbonyl)-$N^6$-(benzyloxycarbonyl)-L-lysine (I) was dissolved in 10 ml of 1,4-dioxane and, while cooling in an ice bath, 280 mg of phosphorus pentachloride was added and the mixture was stirred for about 10 minutes.

After the temperature of the mixture had returned to room temperature, the mixture was stirred for 30 minutes and the solvent and the other evaporating components were distilled off in a water bath at a temperature of 40°C to 50°C.

Thereafter, 10 ml of 1,4-dioxane was again added to the resultant residue and 370 mg of 3,4-dichloroaniline was added at room temperature with stirring. The reaction was completed in one hour. The reaction mixture was then treated in a conventional manner to obtain 380 mg of $N^2$-(1-naphthalenecarbonyl)-$N^6$-(benzyloxycarbonyl)-L-lysine 3,4-dichloroanilide (II) in the form of a powder.

A 200 mg amount of the compound of (II) was treated with 1.0 ml of a 30% hydrogen bromide in acetic acid solution to obtain 120 mg of the desired $N^2$-(1-naphthalenecarbonyl)-L-lysine 3,4-dichloroanilide.

Example 12

Synthesis of $N^2$-(benzoyl-L-lysine 4-benzoylanilide (i.e., Compound No. 123)

A 1.83 g amount of $N^2$-(t-butyloxycarbonyl)-$N^6$-(benzyloxycarbonyl)-L-lysine and 590 mg of triethylamine were dissolved in 15 ml of tetrahydrofuran. While cooling in an ice-salt bath, 530 mg of ethyl chlorocarbonate was added thereto with stirring and, about 20 minutes later, 950 mg of 4-benzoylaniline was added.

After stirring for 2 hours, the mixture was allowed to stand at room temperature for one night. The mixture was then treated in a conventional manner to obtain 2.4 g of $N^2$-(t-butyloxycarbonyl)-$N^6$-(benzyloxycarbonyl)-L-lysine 4-benzoylanilide (I).

A 600 mg amount of the compound (I) was dissolved in 6N-hydrogen chloride in 2 ml of 1,4-dioxane solution and the mixture was stirred at room temperature for about 5 minutes. Then, 2 ml of 1,4-dioxane was added thereto and the mixture was allowed to stand at room temperature for one hour. Thereafter, 10 ml of ethyl ether was added, $N^6$-(benzyloxycarbonyl)-L-lysine 4-benzoylanilide (hydrochloride) (II) was precipitated. The ethyl ether was separated by decantation. After this procedure was repeated several times, the product was recovered by filtration. The compound (I) was dissolved in 6 ml of N,N-dimethylformamide and 340 mg triethylamine was added. The mixture was stirred at room temperature for 5 minutes and 150 mg of benzoyl chloride was added thereto. The mixture was then stirred at room temperature for 5 hours. The resultant mixture was treated in a conventional manner to obtain 400 mg of $N^2$-benzoyl-$N^6$-(benzyloxycarbonyl)-L-lysine 4-benzoylanilide (III).

A 400 mg amount of the compound (III) was treated with 1.0 ml of a 30% hydrogen bromide in acetic acid solution to obtain 180 mg of the desired $N^2$-benzoyl-L-lysine 4-benzoylanilide.

Example 13

Synthesis of $N^2$-(p-toluoyl)-L-lysine 4-benzylpiperidine amide (i.e., Compound No. 122)

A 800 mg amount of $N^2$-(p-toluoyl)-$N^6$-(benzyloxycarbonyl)-L-lysine (I) and 250 mg of N-hydroxy succinimide were dissolved in 15 ml of 1,4-dioxane and, after adding 400 mg of N,N'-dicyclohexyl carbodiimide (DCC) thereto, the mixture was allowewd to stand at a temperature of 5°C to 1°C for one night. The insoluble matter was filtered off. To the filtrate, 350 mg of 4-benzylpiperidine was added and the mixture was allowed to react at a temperature of 10°C for 10 hours with stirring. The resultant reaction mixture was then treated in conventional manner to obtain 910 mg of $N^2$-(p-toluoyl)-$N^6$-(benzyloxycarbonyl)-L-lysine 4-benzylpiperidinamide (II).

A 910 mg amount of the compound (II) was treated with 2.0 ml of a 30% hydrogen bromide in acetic acid solution to obtain 400 mg of the desired $N^2$-(p-toluoyl-L-lysine 4-benzoylpiperidine amide.

Example 14

Synthesis of $N^2$-(6-amino-1-oxo-hexyl)-L-lysine 4-benzylanilide (i.e., Compound No. 112)

A 2.45 g amount of N'-(t-butyloxycarbonyl)-$N^6$-(benzyloxycarbonyl)-L-lysine (I) was dissolved in 10 ml of tetrahydrofuran and 800 mg of triethylamine was then added thereto. While cooling in an ice bath, 800 mg of ethyl chlorocarbonate was added and the mixture was stirred for about 20 minutes. The mixture was suction filtered by using, as a receiver, 790 mg of 4-benzylaniline dissolved in a small amount of tetrahydrofuran. After allowing the stand for one night, the resultant mixture was extracted with ethyl acetate. The extract was treated in a conventional manner to obtain 3.04 g of $N^2$-(t-butyloxycarbonyl)-$N^6$-(benzyloxycarbonyl)-L-lysine 4-benzylanilide (II).

A 2 g amount of 6-benzyloxycarbonyl aminocaproic acid was dissolved in 30 ml of chloroform and,

after adding 1.1 g of thionyl chloride, the mixture was stirred for 30 minutes. The resultant mixture was distilled in vacuo. Then, n-hexane was added to the residue and 2.2 g of 6-benzyloxycarbonyl aminocaproyl chloride (III) was recovered by filtration.

A 10 ml amount of a 6N-hydrogen chloride in dioxane solution was added to 3.04 g of the compound (II) and the mixture was stirred at room temperature for one hour. After adding 10 ml of 1,4-dioxane, the mixture was allowed to stand at room temperature. One hour later, diethyl ether was added to the mixture. The decantation was repeated several times and 30 ml of N,N-dimethylformamide was added. To the mixture, 2.3 g of triethylamine and 2.2 g of the compound (III) were added and the mixture was warmed at a temperature of 40°C. After allowing to stand for one night, the triethylamine hydrochloride was filtered off and the solvent was distilled off. The residue was then extracted with chloroform. The extract was then treated in a conventional manner to obtain 1.8 g of $N^2$-(6-benzyloxycarbonyl amino-1-oxo-hexyl)-$N^6$-(benzyloxycarbonyl)-L-lysine 4-benzylanilide (IV).

A 500 mg amount of the compound (IV) was treated with 1.5 ml of a 30% hydrogen bromide in acetic acid solution to obtain 280 mg of the desired $N^2$-(6-aminohexylcarbonyl)-L-lysine 4-benzylanilide.

Example 15

Synthesis of $N^1$-(4-aminobenzenecarbonyl)-L-lysine 4-benzoyl-anilide (i.e., Compound No. 159)

A 5 g amount of 4-aminobenzoic acid was dissolved in 55 ml of a 2N aqueous sodium hydroxide solution and, while cooling in an ice bath, 6.8 g of benzyloxycarbonyl chloride was added thereto, followed by stirring under ice cooling for 3 hours. The resultant mixture was treated in a conventional manner and to obtain 4.8 g of 4-benzyloxycarbonylaminobenzoic acid (I) by crystallizing from ethyl acetate.

A 0.5 g amount of $N^2$-(t-butyloxycarbonyl)-$N^6$-(benzyloxycarboxyl)-L-lysine 4-benzoylanilide (II) was dissolved in a 6N hydrogen chloride in 1,4-dioxane solution while cooling in an ice bath and 0.4 g of $N^6$-(benzyloxycarboxyl)-L-lysine 4-benzoylanilide hydrochloride was obtained therefrom in the same manner as in Example 14. This product was dissolved in 15 ml of N,N-dimethylformamide and, while cooling in an ice bath, 0.27 ml of triethylamine was added thereto. A 0.39 g amount of the compound (I) was dissolved in chloroform and 0.4 ml of thionyl chloride was added thereto at room temperature. After 5 hours, the chloroform and the other evaporating materials were distilled off. To the residue, 15 ml of N,N-dimethylformamide was added to prepare the solution and this solution was then added to the previously prepared solution.

The N,N-dimethylformamide and the other evaporating components were distilled off and the residue was extracted with ethyl acetate. The extract was treated in a conventional manner to obtain 0.51 g of $N^2$-(4-benzyloxycarbonylamino benzenecarbonyl)-$N^6$-(benzyloxycarbonyl)-L-lysine 4-benzoylanilide (III).

A 83.7 mg amount of the compound (III) was dissolved in 8 ml of water-ethanol and the mixture was subjected to a catalytic reduction. After 14 hours, palladium was filtered off and the filtrate was treated with ethyl ether in a conventional manner to crystallize 39.3 mg of $N^2$-(4-aminobenzenecarbonyl)-L-lysine 4-benzoylanilide (IV).

Example 16

Synthesis of $N^2$-(trans-4-aminomethylcyclohexylcarbonyl)-L-lysine 4-styrylanilide (i.e., Compound No. 146)

A 4.35 g amount of $N^2$-(t-butyloxycarbonyl)-$N^6$-(benzyloxycarbonyl)-L-lysine and 1.39 g of triethylamine were dissolved in 50 ml of tetrahydrofuran. While cooling in an ice-salt bath, 1.24 g of ethyl chlorocarbonate was added with stirring. After about 20 minutes, 2.23 g of 4-aminostylbene was added. After the mixture was stirred for about 2 hours, the resultant mixture was allowed to stand at room temperature for one night. The mixture was then treated in a conventional manner to obtain 4.7 g of $N^2$- (t-butyloxycarbonyl)-$N^6$-(benzyloxycarbonyl)-L-lysine 4-styrylanilide (I).

A 2.0 g amount of the compound (I) was dissolved in 4.8 ml of a 6N-hydrogen chloride in 1,4-dioxane solution and the mixture was stirred at room temperature for about 5 minutes. Furthermore, 4.8 ml of 2,4-dioxane was added thereto and the mixture was allowed to stand at room temperature for one night. Then, 20 ml of ethyl ether was added, $N^6$-(benzyloxycarbonyl)-L-lysine 4-styrylanilide (II) was precipitated. The ethyl ether was removed by decantation. This procedure was repeated several times. The compound (II) was dissolved in 10 ml of N,N-dimethylformamide and 460 mg of triethylamine was added thereto. After the mixture was stirred at room temperature for 5 minutes, 700 mg of trans-4-benzyloxycarbonylamino-methylcyclohexylcarbonyl chloride was added and the mixture was stirred at room temperature for 5 hours. The resultant mixture was treated in a coventional manner to obtain 500 mg of $N^2$-(trans-4-benzyloxycarbonyl cyclohexylcarbonyl)$N^6$-(benzyloxycarbonyl)-L-lysine 4-styrylanilide (III).

A 500 mg amount of the compound (III) was treated with 1.5 ml of a 30% hydrogene bromide in acetic acid solution to obtain 220 mg of the desired N'-trans-4-aminomethylcyclohexylcarbonyl)-L-lysine 4-styrylanilide.

Example 17

Synthesis of $N^2$-(trans-4-aminomethylcyclohexylcarbonyl)-L-lysine 4-acetylanilide (i.e., Compound No. 145)

To 5 ml of a solution of 718 mg of $N^2$-(t-butyloxycarbonyl)-$N^6$-(benzyloxycarbonyl)-L-lysine and 223 mg of tetrahydrofuran dissolved in tetrahydrofuran, 2 ml of a solution of 224 mg of ethyl chlorocarbonate in

tetrahydrofuran was added with stirring while cooling in an ice bath. After about 30 minutes, 280 mg of 4-aminoacetophenone was added. After the ice bath was removed, the mixture was stirred at room temperature and was then allowed to stand for one night. Ice water was added to the reaction mixture and the resultant mixture was extracted with ethyl acetate. The extract was then treated in a conventional manner to obtain 592 mg of $N^2$-(t-butyloxycarbonyl)-$N^6$-(benzyloxycarbonyl)-L-lysine 4-acetylanilide (I).

Then, 3.0 ml of 6N hydrogen chloride in 1,4-dioxane solution is added to 448 mg of the compound (I). After the mixture was stirred at room temperature for 2 hours, the mixture was concentrated in vacuo. Furthermore, toluene was added to the residue and the mixture was concentrated in vacuo. Thus, $N^6$-(benzyloxycarbonyl)-L-lysine-4-acetylanilide hydrochloride (II) was obtained. To this compound (II), 10 ml of a tetrahydrofuran solution containing the mixed acid anhydride of the previously prepared trans-4-(benzyloxycarbonylaminomethyl) cyclohexanecarboxylic acid (III) with ethyl chlorocarbonate and, further, 112 mg of triethylamine were added. The mixture was stirred at room temperature for 4 hours and ice water was then added thereto. The precipitated crystalline substance was recovered by filtration and was thoroughly washed with water. After drying, 328 mg of $N^2$-(trans-4-(benzyloxycarbonylaminomethylcyclohexylcarbonyl)-$N^6$-(benzyloxycarbonyl)-L-lysine 4- acetylanilide (IV) was obtained.

A 200 mg amount of the compound (IV), 100 mg of 10% Pd-carbon powder, and 4 ml of cyclohexane were dissolved in 20 ml of ethanol and the resultant solution was vigorously stirred for 2 hours. The 10% Pd-carbon powder was filtered off and the filtrate was concentrated in vacuo. The residue was crystallized from ethyl acetate to obtain 57 mg of the desired $N^2$-(trans-4-aminomethylcyclohexylcarbonyl)-L-lysine 4-acetylanilide.

The inhibition activities of the present compounds and the control compounds are evaluated as follows.

(1) Evaluation of Antiplasmin Activity

(i) Determination of prevention activity for fibrin decomposition

0.18 M borate-physiological salt buffer solution (pH = 7.4) to make the total volme to 600 µl. To this buffer solution, 200 µl of a 0.2% bovin fibrinogen, 100 µl of a 0.3 cassein unit/ml human plasma solution, and 100 µl of a 50 unit/ml bovine thrombin solution, all dissolved in the above-mentioned buffer, are added at a temperature of 37°C in a constant temperature bath. Then, the dissolution time of the fibrin mass formed above is determined. Thus, the concentration $I_{50}$ of the inhibitor sample (i.e., 50% inhibition concentration), at which the dissolution time obtained in the absence of the inhibitor (i.e., about 5 minutes) is extended twice, is determined.

(ii) Determination of prevention activity for S—2251 decomposition

An inhibitor sample is dissolved in a 0.05 M Tris-hydrochloric acid buffer solution (pH = 7.4) to make the total volume to 400 µl. To this solution, 50 µl of a 3 mM S—2251 solution is added and the mixture is incubated at a temperature of 37°C for 5 minutes in a constant temperature bath. Then, 50 µl of a 0.2 casein unit/ml human plasmin is added and the mixture is incubated at a temperature of 37°C for 4 minutes. Thereafter, the reaction is stopped by adding 50 µl of 50% acetic acid.

The absorbance of p-nitroaniline formed in the reaction mixture is determined at 405 nm. Thus, the concentration $I_{50}$ of the inhibitor sample, at which the absorbance is one half (i.e., 1/2) of that obtained in the absence of the inhibitor, is determined.

(iii) Determination of prevention activity for fibrinogen

An inhibitor sample is dissolved in a 0.18 M borate-physiological salt buffer solution (pH = 7.4) to make the total volume to 400 µl. To this solution, 500 µl of a 0.4% bovine fibrinogen solution and 100 µl of a 1 casein unit/ml human plasmin solution, all dissolved in the above-mentioned buffer are added at a temperature of 37°C in a constant temperature bath. After the mixture is allowed to stand at a temprature of 37°C for 10 minutes, 3800 µl of the above-mentioned buffer containing (3.2 mM of tranexamic acid and 200 µl of a 50 unit/ml bovine thrombin solution are added to terminate the reaction. The mixture is incubated at a temperature of 37°C for 15 minutes to precipitate the fibrin. The fibrin mass thus precipitated is adhered to or wound around a glass rod and is then washed with water. The amount of the remaining fibrinogen is determined according to a tyrosine coloring method using a phenol reagent (see J. Biol. Chem., *73*, 627 (1927). From the amount of the remaining fibrinogen thus determined, the amount of decomposed fibrinogen is calculated. Thus, the concentration $I_{50}$ of the inhibitor sample, at which the amount of decomposed fibrinogen is one half (i.e., 1/2) of that obtained in the absence of the inhibitor sample, is determined.

(2) Evaluation of Antithrombin Activity

(i) Determination of inhibition activity against fibrin formation

An inhibitor sample is dissolved in a 0.18 M borate-physiological salt buffer solution (pH = 7.4) to make the total volume to 500 µl. To this solution, 400 µl of a 0.2% bovine fibrinogen solution and 100 µl of a 4 unit/ml bovine thrombin solution are added at a temperature of 37°C in a constant temperature bath. Thus, a coagulation time is determined. The inhibitor concentraion $I_{50}$, at which the coagulation time obtained in

the absence of the inhibitor is extended twice, is determined.

(ii) Determination of prevention activity for S—2238 decomposition

An inhibitor sample is dissolved in a 0.05 M Tris-hydrochloric acid buffer solution (pH = 8.3) to make a total volume to 400 µl. To this solution, 50 µl of a 0.2% mM S—2238 solution is added and the mixture is incubated at a temperature of 37°C for 5 minutes in a constant temperature bath. Then, 50 µl of a 0.2 unit/ml bovine thrombin solution is added thereto and the absorbance, at 405 nm, of the p-nitroaniline formed per minute is determined at a temperature of 37°C by using the so-called initial velocity method. Thus, the concentration $I_{50}$ of the inhibitor sample at which the absorbance is one half (i.e., 1/2) of that obtained in the absence of the inhibitor, is determined.

(3) Evaluation of Antitrypsin Activity

Determination of inhibition activity against S—2238 decomposition

An inhibitor sample is dissolved in a 0.05 M Tris-imidazole buffer solution (pH = 8.1) and 125 µl of a 1 mM S—2238 solution is added to make the total volume to 1.20 ml. The mixture is incubated at a temperature of 37°C for 5 minutes in a constant temperature bath. To this mixture, 0.05 ml of bovine trypsin is added and the absorbance, at 405 nm, of the p-nitroaniline formed per minute is determined at a temperature of 37°C by using the so-called initial velocity method. Thus, the concentration $I_{50}$ of the inhibitor sample, at which the absorbance is one half (i.e., 1/2) of that obtained in the absence of the inhibitor, is determined.

(4) Evaluation of Anti-Plasma Kallikrein Activity

Determination of prevention activity for S—2302 decomposition

An inhibitor sample is dissolved in a 0.05 M Tris-hydrochloric acid buffer solution (pH = 7.8) to make the total volume to 400 µl. To this solution, 50 µl of a 2 mM S—2302 solution is added and the mixture is incubated at a temperature of 37°C for 5 minutes in a constant temperature bath. Then, 50 µl of a 0.12 unit/ml human plasma kallikrein is added and the mixture is incubated at a temperature of 37°C for 5 minutes. Thereafter, 50 µl of 50% acetic acid is added to terminate the reaction. The absorbance of the p-nitroaniline formed in the reaction mixture is measured at 405 nm. Thus, the concentration $I_{50}$ of the inhibitor sample, at which the absorbance is one half (i.e., 1/2) of that obtained in the absence of the inhibitor sample, is determined.

(5) Evaluation of Antiurokinase Activity

Determination of prevention activity for S—2444 decomposition

An inhibitor sample is dissolved in a 0.05 M Tris-hydrochloric acid buffer solution (pH = 8.8) to make the total volume to 400 µl. To this solution, 50 µl of a 1 mM S—2444 solution is added and the mixture is incubated at a temperature of 37°C for 5 minutes in a constant temperature bath. Then, 50 µl of a 500 unit/ml human urokinase is added and the mixture is incubated at a temperature of 37°C for 5 minutes. Thereafter, 50 µl of 50% acetic acid is added to terminate the reaction. The absorbance of the p-nitroaniline formed in the reaction mixture is measured at 405 nm. Thus, the concentration $I_{50}$ of the inhibitor sample, at which the absorbance is one half (i.e., 1/2) of that obtained in the absence of the inhibitor, is determined.

The results are shown in Tables II to V.

Table II (List of Known Compounds)

| Compound No. | Compound |
|---|---|
| 1 | $H_2NCH_2$ 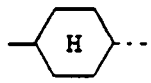 $CO_2H$ (t-AMCHA) |
| 2 | $H_2N(CH_2)_5CO_2H$ (EACA) |
| 3 | H-Lys-OH |
| 4 | 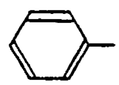 $SO_2$-Lys-$NH_2$ |
| 5 | $CH_3$ 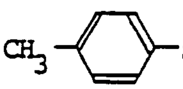 $SO_2$-Lys-OH |
| 6 | $CH_3$ 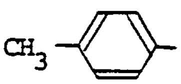 $SO_2$-Arg-$OCH_3$ (TAME) |
| 7 | $H_2N(CH_2)_5CO$-Lys-OH |
| 8 | 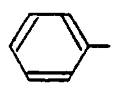 CO-Lys-$NH_2$ |

Table III (Evaluation Results of Known Compounds)

$I_{50}$ (μM)

| Compound No. | Plasmin | | | Thrombin | | Trypsin | Plasma Kallikrein | Urokinase |
|---|---|---|---|---|---|---|---|---|
| | S-2251 | Fibrin | Fibrinogen | S-2238 | Fibrinogen | S-2238 | S-2302 | S-2444 |
| 1 | 75,000 | 60 | 9,500 | 1,000*1 | 1,000*1 | 1,000*1 | 1,000*1 | 1,000*1 |
| 2 | 180,000 | 200 | - | - | - | - | - | - |
| 3 | 50,000 | 9,000 | - | - | - | - | - | - |
| 4 | 1,000*1 | 1,000*1 | - | 1,000*1 | 1,000*1 | 150*1 | 1,000*1 | 1,000*1 |
| 5 | 1,400 | 1,000 | - | - | - | - | - | - |
| 6 | 3,100 | 1,000 | - | - | - | - | - | - |
| 7 | 1,000*1 | 1,000*2 | - | 1,000*1 | 1,000*1 | 200*1 | 1,000*1 | 1,000*1 |
| 8 | 1,000*1 | 1,000*1 | - | 1,000*1 | 1,000*1 | 300*1 | 1,000*1 | 1,000*1 |

*1: 0% Inhibition
*2: 19% Inhibition

Table IV (Evaluation Results of Present Compounds)

| Compound No. | $I_{50}$ (M) | | |
|---|---|---|---|
| | S-2251 | Fibrin | Fibrinogen |
| 3 | 7.0 x $10^{-4}$ | 7.8 x $10^{-4}$ | 9.0 x $10^{-4}$ |
| 7 | 3.5 x $10^{-4}$ | 3.0 x $10^{-4}$ | - |
| 8 | 2.5 x $10^{-4}$ | 1.4 x $10^{-4}$ | - |
| 10 | 1.7 x $10^{-3}$ | 3.1 x $10^{-4}$ | - |
| 11 | 3.9 x $10^{-4}$ | 7.5 x $10^{-5}$ | 8.0 x $10^{-5}$ |
| 18 | 6 x $10^{-4}$ | 3.5 x $10^{-4}$ | - |
| 20 | 2 x $10^{-3}$ | 1 x $10^{-3}$ | - |
| 21 | 3 x $10^{-4}$ | 1.5 x $10^{-4}$ | - |
| 22 | 6.5 x $10^{-4}$ | 6.0 x $10^{-4}$ | - |
| 29 | 4.8 x $10^{-4}$ | 3.4 x $10^{-4}$ | - |
| 31 | 7.8 x $10^{-4}$ | 1.5 x $10^{-4}$ | - |
| 32 | 6 x $10^{-4}$ | 4.3 x $10^{-4}$ | - |
| 33 | 6.5 x $10^{-4}$ | 5.4 x $10^{-4}$ | - |
| 35 | 3.7 x $10^{-4}$ | 1.9 x $10^{-4}$ | - |
| 36 | 1.4 x $10^{-4}$ | 1:5 x $10^{-4}$ | 2.0 x $10^{-4}$ |
| 37 | 2.0 x $10^{-3}$ | 7.3 x $10^{-4}$ | - |
| 40 | 5.9 x $10^{-4}$ | 4.4 x $10^{-4}$ | - |
| 42 | 2.3 x $10^{-4}$ | 1 4 x $10^{-4}$ | - |
| 48 | 1.3 x $10^{-4}$ | 7 4 x $10^{-5}$ | - |
| 53 | 6.5 x $10^{-4}$ | 4 5 x $10^{-4}$ | - |
| 54 | 2 x $10^{-4}$ | 1 x $10^{-4}$ | - |
| 56 | 2 x $10^{-4}$ | 3.5 x $10^{-5}$ | - |
| 59 | 6.9 x $10^{-5}$ | 6 .1 x $10^{-5}$ | - |

Table IV (Continued)

| Compound No. | $I_{50}$ (M) | | |
|---|---|---|---|
| | S-2251 | Fibrin | Fibrinogen |
| 60 | $1.5 \times 10^{-4}$ | $3.4 \times 10^{-5}$ | - |
| 62 | $1.6 \times 10^{-4}$ | $2.7 \times 10^{-5}$ | - |
| 64 | $3.3 \times 10^{-5}$ | $5.0 \times 10^{-5}$ | - |
| 65 | $1.5 \times 10^{-3}$ | $8.5 \times 10^{-4}$ | - |
| 67 | $5.2 \times 10^{-5}$ | $5.5 \times 10^{-5}$ | - |
| 68 | $4.4 \times 10^{-4}$ | $2.2 \times 10^{-4}$ | - |
| 69 | $1.6 \times 10^{-4}$ | $1.2 \times 10^{-4}$ | - |
| 70 | $7.3 \times 10^{-5}$ | $3.4 \times 10^{-5}$ | - |
| 72 | $2.0 \times 10^{-4}$ | $2.3 \times 10^{-4}$ | - |
| 73 | $4.4 \times 10^{-5}$ | - | - |
| 74 | $7 \times 10^{-5}$ | $1.0 \times 10^{-4}$ | - |
| 75 | $3.7 \times 10^{-5}$ | $7.5 \times 10^{-5}$ | $5.0 \times 10^{-5}$ |
| 76 | $4.3 \times 10^{-5}$ | $1.2 \times 10^{-4}$ | - |
| 77 | $4.6 \times 10^{-4}$ | $1.6 \times 10^{-4}$ | - |
| 78 | $3.8 \times 10^{-5}$ | $1.9 \times 10^{-4}$ | - |
| 80 | $4.2 \times 10^{-5}$ | $5.1 \times 10^{-5}$ | $8.0 \times 10^{-5}$ |
| 82 | $1.3 \times 10^{-4}$ | $1.0 \times 10^{-4}$ | - |
| 83 | $8.8 \times 10^{-5}$ | $1.1 \times 10^{-4}$ | - |
| 85 | $4.5 \times 10^{-4}$ | $1.7 \times 10^{-4}$ | - |
| 89 | $3.2 \times 10^{-5}$ | $2.9 \times 10^{-5}$ | - |
| 90 | $2.7 \times 10^{-4}$ | $2.5 \times 10^{-4}$ | - |
| 91 | $2.5 \times 10^{-4}$ | $6.1 \times 10^{-5}$ | - |
| 93 | $6.8 \times 10^{-4}$ | $3.5 \times 10^{-4}$ | - |

Table IV (Continued)

| Compound No. | $I_{50}$ (M) | | |
|---|---|---|---|
| | S-2251 | Fibrin | Fibrinogen |
| 94 | $2.0 \times 10^{-4}$ | $3.2 \times 10^{-5}$ | - |
| 95 | $1.5 \times 10^{-4}$ | $4 \times 10^{-5}$ | - |
| 97 | $3 \times 10^{-4}$ | $5 \times 10^{-4}$ | - |
| 104 | $1.0 \times 10^{-4}$ | $3.1 \times 10^{-5}$ | $4.0 \times 10^{-5}$ |
| 108 | $4.1 \times 10^{-5}$ | $2.0 \times 10^{-5}$ | $4.0 \times 10^{-5}$ |
| 109 | $1.2 \times 10^{-4}$ | $6.8 \times 10^{-6}$ | - |
| 111 | $2.0 \times 10^{-4}$ (0% Inhibition) | $2.0 \times 10^{-4}$ (0% Inhibition) | - |
| 116 | $5.0 \times 10^{-4}$ (41% Inhibition) | $5.3 \times 10^{-4}$ | - |
| 119 | $5.5 \times 10^{-4}$ | $5.0 \times 10^{-4}$ | - |
| 120 | $8.0 \times 10^{-4}$ | $7.5 \times 10^{-4}$ | - |
| 121 | $6.7 \times 10^{-4}$ | $1.2 \times 10^{-3}$ | - |
| 124 | $1.6 \times 10^{-4}$ | $1.9 \times 10^{-4}$ | - |
| 128 | $1.6 \times 10^{-4}$ | $5.0 \times 10^{-4}$ | - |
| 132 | $2.4 \times 10^{-5}$ | $7.5 \times 10^{-5}$ | - |
| 141 | $1.5 \times 10^{-5}$ | $6.1 \times 10^{-6}$ | $1.3 \times 10^{-5}$ |
| 142 | $2.8 \times 10^{-4}$ | $9.3 \times 10^{-5}$ | - |
| 145 | $3.9 \times 10^{-5}$ | $9.3 \times 10^{-6}$ | $1.9 \times 10^{-5}$ |
| 146 | $1.8 \times 10^{-4}$ | $3.1 \times 10^{-4}$ | - |
| 154 | $1.2 \times 10^{-5}$ | $4.5 \times 10^{-5}$ | - |
| 156 | $1.6 \times 10^{-5}$ | $1.7 \times 10^{-5}$ | $3.6 \times 10^{-5}$ |
| 158 | $1.0 \times 10^{-4}$ | $1.6 \times 10^{-4}$ | - |

Table V (Evaluation Results of Present Compounds)

| Compound No. | Thrombin | | Trypsin | Plasma Kallikrein | Urokinase |
|---|---|---|---|---|---|
| | S-2238 | Fibrinogen | S-2238 | S-2302 | S-2444 |
| 62 | $5.0 \times 10^{-5}$ (0% Inhibition) | $5.0 \times 10^{-5}$ (0% Inhibition) | $1.5 \times 10^{-4}$ | - | - |
| 80 | $1.0 \times 10^{-4}$ (24% Inhibition) | $1.0 \times 10^{-4}$ (0% Inhibition) | $3.3 \times 10^{-4}$ | - | - |
| 89 | $2.0 \times 10^{-5}$ (22% Inhibition) | $4.0 \times 10^{-5}$ (0% Inhibition) | $3.5 \times 10^{-4}$ (0% Inhibition) | - | - |
| 104 | $1.0 \times 10^{-4}$ (0% Inhibition) | $1.0 \times 10^{-4}$ (0% Inhibition) | $2.4 \times 10^{-5}$ | $7.6 \times 10^{-5}$ | $1.1 \times 10^{-4}$ |
| 108 | $1.0 \times 10^{-3}$ (23% Inhibition) | $1.0 \times 10^{-3}$ (0% Inhibition) | $6.4 \times 10^{-6}$ | $1.5 \times 10^{-5}$ | $8.5 \times 10^{-6}$ |
| 109 | $4.0 \times 10^{-4}$ (0% Inhibition) | $3.0 \times 10^{-4}$ (0% Inhibition) | $1.2 \times 10^{-5}$ | $2.9 \times 10^{-5}$ | $4.5 \times 10^{-5}$ |
| 111 | - | - | - | $2.0 \times 10^{-4}$ (0% Inhibition) | - |
| 116 | $5.0 \times 10^{-5}$ (0% Inhibition) | $1.0 \times 10^{-3}$ (0% Inhibition) | $5.0 \times 10^{-5}$ | $5.0 \times 10^{-5}$ | $1.3 \times 10^{-4}$ |
| 141 | $5.0 \times 10^{-4}$ (0% Inhibition) | $1.0 \times 10^{-4}$ (0% Inhibition) | $1.5 \times 10^{-6}$ | $8.5 \times 10^{-5}$ | $1.7 \times 10^{-5}$ |

## Claims

1. A lysine derivative having the general formula:

A-Y-Lys-B (L-form) (A)

where A represents

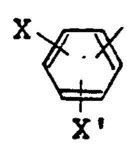 , 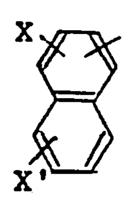 , 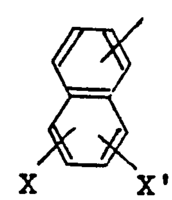 ,

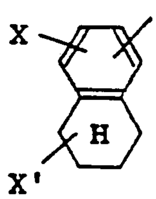 , 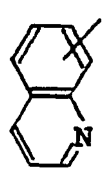 , 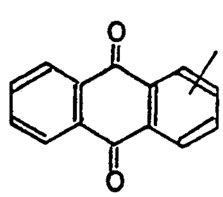 , 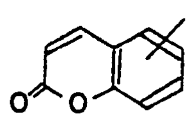 ,

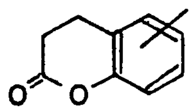, 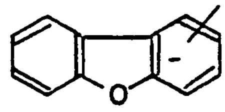, 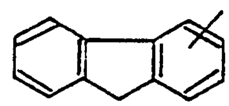,

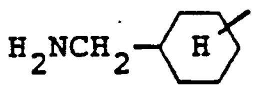, $H_2N(CH_2)_{n_1}-$, 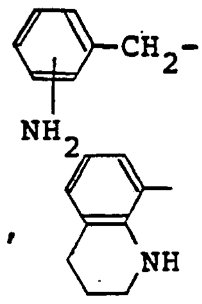,

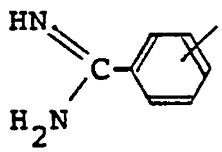, 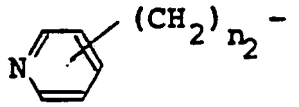, ,

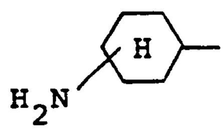, or 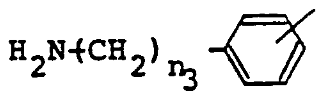;

wherein X and X′ independently represent hydrogen, halogen, alkyl, cycloalkyl, alkoxy, aryloxy, dialkylamino, alkylcarbonylamino, arylcarbonylamino, and $n_1$ is an integer of 3 to 6, $n_2$ is an integer of 1 to 3, and $n_3$ is an integer of 0 to 3;

Y represents $SO_2$ or CO;

-Lys- represents

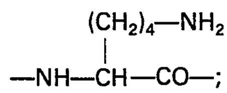

B represents $NR^1R^2$, NZW, or tetrahydroquinolyl, wherein $R^1$ and $R^2$ independently represents hydrogen provided that both $R^1$ and $R^2$ cannot be hydrogen at the same time; alkyl substituted with carboxyl, alkoxycarbonyl, phenyl, hydroxyphenyl, or benzoyl; cycloalkyl which may be substituted with arylcarbonyl; cycloalkyl-alkyl which may be substituted with carboxyl, arylcarbonyl, or aralkyloxycarbonyl; phenyl which may be substituted with halogen, nitro, cyano, trifluoromethyl, alkyl, alkoxy, alkoxycarbonyl, alkoxycarbonylalkyl, phenylalkyl which may be further substituted with dialkylamino, alkylcarbonyl, phenylalkenyl which may be further substituted with dialkylamino, phenoxy, phenylcarbonyl which may be further substituted with an amino, dialkylamino, alkoxycarbonyl, or nitro group, pyridylmethyl, phenyl hydroxyalkyl, alkylsulfonyl, or alkoxycarbonyl alkylaminocarbonyl; coumaryl which may be sustituted with alkyl; quinolyl; adamantyl; norbornyl; or tetrahydronaphthyl; and

Z is

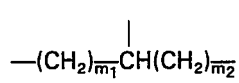

or

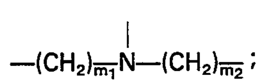

W is hydrogen; hydroxyl; carboxyl; aminocarbonyl; alkyl; alkoxycarbonyl; phenyl; phenylalkyl which may be substituted with dialkylamino; or phenyl-carbonyl which may be substituted with alkoxycarbonyl or tetrahydroquinolyl; and

$$m_1 + m_2 = 3 \text{ or } 4;$$

or the pharmaceutically acceptable salt thereof.

2. A lysine derivative as claimed in claim 1, wherein the pharmaceutically acceptable salt is at least one salt selected from the group consisting of hydrochloride, hydrobromide, sulfate, nitrate, phosphate, oxalate, succinate, malate, citrate, lactate, benzene sulfonate, toluene, sulfonate, and methane sulfonate.

3. A proteinase inhibitor comprising as an essential component the lysine derivative of claim 1 or the pharmaceutically acceptable salt thereof.

4. A proteinase inhibitor as claimed in claim 3, wherein the pharmaceutically acceptable salt is at least one salt selected from the group consisting of hydrochloride, hydrobromide, sulfate, nitrate, phosphate, oxalate, succinate, malate, citrate, lactate, benzene sulfonate, toluene sulfonate, and methane sulfonate.

**Patentansprüche**

1. Lysinderivat der allgemeinen Formel:

A-Y-Lys-B (L-Form) (A)

worin A bedeutet

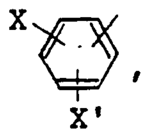, 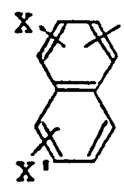, 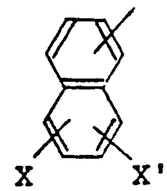,

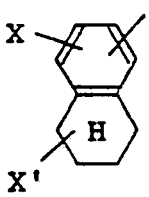, 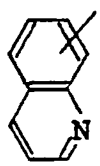, 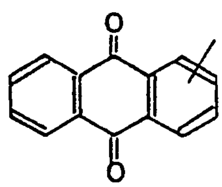, 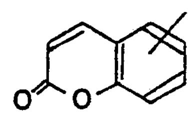,

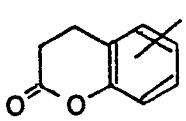, 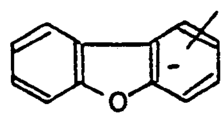, 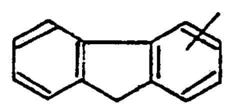,

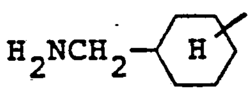, $H_2N(CH_2)_{n_1}-$, 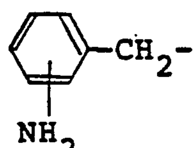,

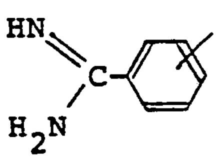, 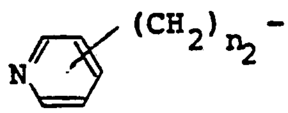, 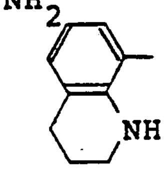,

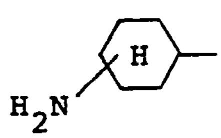, oder 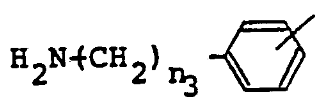 ;

worin X und X′ unabhängig voneinander Wasserstoff, Halogen, Alkyl, Cycloalkyl, Alkoxy, Aryloxy, Dialkylamino, Alkylcarbonylamino, Arylcarbonylamino darstellen und $n_1$ eine ganze Zahl von 3 bis 6, $n_2$ eine ganze Zahl von 1 bis 3 und $n_3$ ganze Zahl von 0 bis 3 ist;

Y für $SO_2$ oder CO steht;

-Lys- für

$$\begin{array}{c} (CH_2)_4{-}NH_2 \\ | \\ {-}NH{-}CH{-}CO{-} \end{array}$$

steht;

B für $NR^1R^2$, NZW oder Tetrahydrochinolyl steht, wobei $R^1$ und $R^2$ unabhängig voneinander bedeuten: Wasserstoff, mit der Maßgabe, daß nicht beide $R^1$ und $R^2$ gleichzeitig für Wasserstoff stehen, mit Carboxyl, Alkoxycarbonyl, Phenyl, Hydroxyphenyl oder Benzoyl substituiertes Alkyl; Cycloalkyl, das mit Arylcarbonyl

substituiert sein kann; Cycloalkyl-alkyl, das mit Carboxyl, Arylcarbonyl oder Aralkyloxycarbonyl substituiert sein kann; Phenyl, das substituiert sein kann mit Halogen, Nitro, Cyan, Trifluormethyl, Alkyl, Alkoxy, Alkoxycarbonyl, Alkoxycarbonylalkyl, Phenylalkyl, welches wiederum mit Dialkylamino, Alkylcarbonyl substituiert sein kann, Phenylalkenyl, welches wiederum substituiert sein kann mit Dialkylamino, Phenoxy, Phenylcarbonyl, welches wiederum substituiert sein kann mit einer Amino-, Dialkylamino-, Alkoxycarbonyl- oder Nitrogruppe, Pyridylmethyl, Phenylhydroxyalkyl, Alkylsulfonyl, oder Alkoxycarbonylalkylaminocarbonyl; Cumaryl, welches mit Alkyl substituiert sein kann; Chinolyl; Adamantyl; Norbornyl; oder Tetrahydronaphthyl; und

Z bedeutet

$$\overset{|}{-(CH_2)_{m_1}CH(CH_2)_{m_2}}$$

oder

$$-(CH_2)_{m_1}\overset{|}{N}-(CH_2)_{m_2};$$

W für Wasserstoff, Hydroxyl, Carboxyl, Aminocarbonyl, Alkyl, Alkoxycarbonyl, Phenyl, Phenylalkyl, das mit Dialkylamino substituiert sein kann, oder Phenylcarbonyl, das mit Alkoxycarbonyl oder Tetrahydrochinolyl substituiert sein kann, steht, und

$$m_1 + m_2 = 3 \text{ oder } 4;$$

oder dessen pharmazeutisch brauchbares Salz.

2. Lysinderivat gemäß Anspruch 1, wobei das pharmazeutisch brauchbare Salz mindestens ein aus der Gruppe Hydrochlorid, Hydrobromid, Sulfat, Nitrat, Phosphat, Oxalat, Succinat, Malat, Citrat, Lactat, Benzolsulfonat, Toluolsulfonat und Methansulfonat ausgewähltes Salz ist.

3. Proteinaseinhibitor, enthaltend als wesentliche Komponente das Lysinderivat gemäß Anspruch 1 oder dessen pharmazeutisch brauchbares Salz.

4. Proteinaseinhibitor gemäß Anspruch 3, wobei das pharmazeutisch brauchbare Salz mindestens ein aus der Gruppe Hydrochlorid, Hydrobromid, Sulfat, Nitrat, Phosphat, Oxalat, Succinat, Malat, Citrat, Lactat, Benzolsulfonat, Toluolsulfonat und Methansulfonat ausgewähltes Salz ist.

## Revendications

1. Dérivé de lysine répondant à la formule générale:

A-Y-Lys-B (formel) (A)

dans la A représente

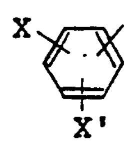 , 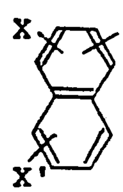 , 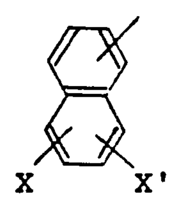 ,

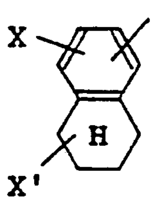 , 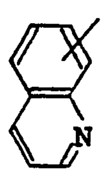 , 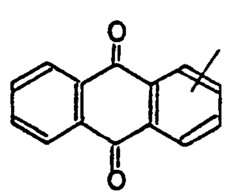 , 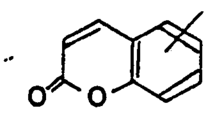 ,

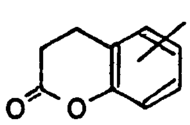 , 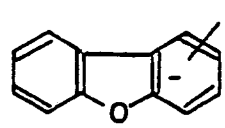 , 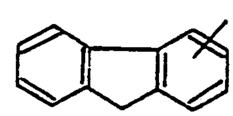 ,

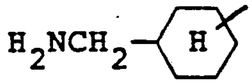, $H_2N(CH_2)_{n_1}-$, 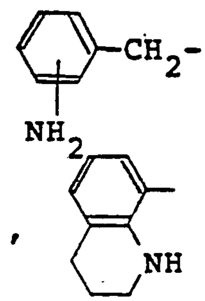,

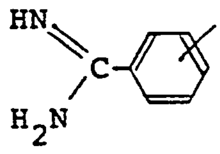, 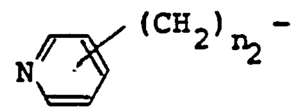,

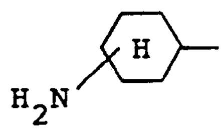, ou 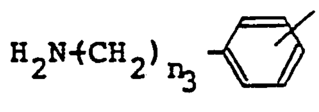;

ou X et X' représentent independamment l'hydrogène, un halogène, un groupe alkyle, cycloalkyle, alcoxy, aryloxy, dialkylamino, alkylcarbonylamino, arylcarbonylamino et $n_1$ est un nombre entier de 3 à 6, $n_2$ est un nombre entier de 1 à 3 et $n_3$ est un nombre entier de 0 à 3;

Y représente $SO_2$ ou CO;

-Lys- représente

$$-NH-\underset{\displaystyle |}{\overset{(CH_2)_4-NH_2}{CH}}-CO-CO-;$$

B représente $NR^1R^2$, NZW ou un groupe tétrahydroquinolyle, où $R_1$ et $R_2$ représentent indépendamment l'hydrogène, etant entendu que $R_1$ et $R_2$ ne peuvent pas représenter l'hydrogène tous les deux en même temps; un groupe substitué par un groupe carboxyle, alcoxycarbonyle, phényle, hydroxyphényle ou benzoyle; un groupe cycloalkyle pouvant être substitué par un groupe arylcarbonyle; un groupe cycloalkyl-alkyle pouvant être substitué par un groupe carboxyle, arylcarbonyle ou aralkyloxycarbonyle; un groupe phényle qui peut être substitué par un groupe halogène, nitro, cyano, trifluorométhyle, alkyle, alcoxy, alcoxycarbonyle, alcoxycarbonylalkyle, phénylalkyle pouvant être substitué a son tour par un groupe dialkylamino, alkylcarbonyle, phénylacényle qui peut être substitué à son tour par un groupe dialkylamino, phénoxy, phénylcarbonyle, qui peut être substitué a son tour par un groupe amino, dialkylamino, alcoxycarbonyle ou nitro, pyridylmethyl, phénylhydroxyalkyle, alkylsulfonyle ou alcoxycarbonyl-alkylaminocarbonyle; un groupe coumaryle qui peut être substitue par un groupe alkyle; un groupe quinolyle; un groupe adamantyle; un groupe norbornyle; ou un groupe tetrahydronaphtyle; et

Z est

$$-(CH_2)_{m_1}-\overset{|}{C}H(CH_2)_{m_2}$$

ou

$$-(CH_2)_{m_1}-\overset{|}{N}-(CH_2)_{m_2};$$

W représente l'hydrogéne; un groupe hydroxyle; un groupe carboxyle; un groupe aminocarbonyle; un groupe alkyle; un groupe alcoxycarbonyle; un groupe phényle; un groupe phénylalkyle qui peut être substitué par un groupe dialkylamino; ou un groupe phényl-carbonyle qui peut être substitué par un groupe alcoxycarbonyle ou tétrahydroquinolyle; et

$$m_1 + m_2 = 3 \text{ ou } 4;$$

ou un de ses sels pharmaceutiquement acceptables.

2. Dérivé de lysine selon la revendication 1, dans lequel le sel pharmaceutiquement acceptable est au moins un sel choisi dans le groupe constitué par les chlorhydrates, bromhydrates, sulfates, nitrates, phosphates, oxalates, succinates, malates, citrates, lactates, benzenesulfonates, toluenesulfonates et methanesulfonates.

3. Inhibiteur de protéinase comprenant, comme composé principal, le dérivé de lysine selon la revendication 1, ou son sel pharmaceutiquement acceptable.

4. Inhibiteur de protéinase selon la revendication 3, dans lequel le sel pharmaceutiquement acceptable est au moins un sel choisi dans le groupe constitué par les chlorhydrates, bromhydrates, sulfates, nitrates, phosphates, oxalates, succinates, malates, citrates, lactates, benzènesulfonates, toluènesulfonates et méthanesulfonates.